# EUROPEAN PATENT SPECIFICATION

(11) **EP 1 787 122 B1**
(45) Date of publication and mention of the grant of the patent: **06.10.2010**
(21) Application number: 05770407.4
(22) Date of filing: 09.08.2005
(51) Int. Cl.: G01N 33/574

(54) **NON-SMALL CELL LUNG CANCER-RELATED GENE, ANLN, AND ITS INTERACTIONS WITH RhoA**
GEN ANLN IN VERBINDUNG MIT NICHT-KLEINZELLIGEM LUNGENKREBS UND SEINE WECHSELWIRKUNGEN MIT RhoA
GENE RELATIF AU CANCER DU POUMON NON A PETITES CELLULES, ANLN, ET SES INTERACTIONS AVEC LE RhoA

(30) Priority: 10.08.2004 US 600561 P
(43) Date of publication of application: 23.05.2007
(73) Proprietor: Oncotherapy Science, Inc., Kawasaki-shi Kanagawa 213-0012 (JP)
(72) Inventor: NAKAMURA, Yusuke, Bunkyo-ku, Tokyo 113-8654 (JP); DAIGO, Yataro, Bunkyo-ku, Tokyo 113-8654; (JP); NAKATSURU, Shuichi, Kawasaki-shi, Kanagawa 213-0012 (JP)
(74) Representative: Vossius & Partner
(86) International application number: PCT/JP2005/014887
(87) International publication number: WO 2006/016697

(56) References cited:
- WO-A-2004/031413
- GIANSANTI MARIA GRAZIA ET AL: "Genetic dissection of meiotic cytokinesis in Drosophila males." MOLECULAR BIOLOGY OF THE CELL. MAY 2004, vol. 15, no. 5, May 2004 (2004-05), pages 2509-2522, XP002356183 ISSN: 1059-1524
- OEGEMA K ET AL: "Functional analysis of a human homologue of the Drosophila actin binding protein anillin suggests a role in cytokinesis." THE JOURNAL OF CELL BIOLOGY, vol. 150, no. 3, 7 August 2000 (2000-08-07), pages 539-551, XP002356184 ISSN: 0021-9525
- FIELD C M ET AL: "Anillin, a contractile ring protein that cycles from the nucleus to the cell cortex." THE JOURNAL OF CELL BIOLOGY. OCT 1995, vol. 131, no. 1, October 1995 (1995-10), pages 165-178, XP002356185 ISSN: 0021-9525

## Description

### FIELD OF THE INVENTION

The present method relates to the discovery that *ANLN,* a cancer specific gene up-regulated in non-small cell lung cancer (*see* PCT Publication No. WO 2004/031413), interacts with RhoA, a member of the Ras homology family of small GTPases involved in tumorigenesis.

### BACKGROUND OF THE INVENTION

Lung cancer is one of the most common causes of cancer death worldwide, and non-small cell lung cancer (NSCLC) accounts for nearly 80% of those cases (Greenlee, R.T. et al. CA. Cancer J. Clin. 51:15-36 (2001)). Although many genetic alterations involved in development and/or progression of lung cancer have been reported, the precise molecular mechanism remains unclear (Sozzi, G., Eur. J. Cancer. 37:63-73 (2001)). Newly developed cytotoxic agents have emerged to offer multiple therapeutic choices for patients with advanced NSCLC, but each of the new regimens can provide only modest survival benefits compared with cisplatin-based therapies (Schiller, J.H. et al. N. Engl. J. Med. 346(2):92-98 (2002); Kelly, K. et al. J. Clin. Oncol. 19(13):3210-3218 (2001)). Hence, novel therapeutic strategies, such as development of molecular-targeted agents and antibodies as well as cancer vaccines are eagerly awaited.

Systematic analysis of the expression levels of thousands of genes using a cDNA microarray is an effective approach to identify a set of molecules involved in pathways of carcinogenesis (Kikuchi, T. et al. Oncogene 22(14):2192-2205 (2003); Kakiuchi, S. et al. Mol. Cancer Res. 1:485-499 (2003); Zembutsu, H. et al. Int. J. Oncol. 23:29-39 (2003); Suzuki, C. et al. Cancer Res. 63(21):7038-7041 (2003)), some of which can be candidate targets for development of novel anti-cancer drugs and tumor markers.

The present invention addresses the need in the art for such target molecules. In particular, to isolate novel molecular targets for diagnosis and treatment of NSCLC, the present inventors performed genome-wide expression profile analysis of NSCLC coupled with pure purification of tumor cells from 37 cancer tissues by laser-capture microdissection. In the course of those studies, it was observed that a gene encoding a human homologue of anillin, a Drosophila actin binding protein (ANLN), was over-expressed commonly in primary NSCLCs. ANLN is reported to be essential for the formation or organization of actin cables in the cleavage furrow and to play an important role in cytokinesis (Oegema, K. et al. J. Cell Biol. 150(3):539-551 (2000)). Differential gene expression linked to non-small cell lung cancer has been described previously. *See, e.g.,* PCT Publication No. WO 2004/031413.

Small guanosine triphosphatases (GTPases) play an important role in regulation and coordination of the remodeling of the cytoskeleton. Among the Ras homology family members (RHO) in mammalian cells, RhoA, Ras-related C3 botulinum toxin substrate 1 (RAC1), and cell division cycle 42 (CDC42) have been extensively studied for their biological functions. RhoA regulates a signal transduction pathway linking plasma membrane receptors to the assembly of focal adhesions and the formation of actin stress fibers through the recruitment and activation of its effectors, mDia, Rho-associated, coiled-coil containing protein kinase 1 (ROCK1), and ROCK2 (Ridley, A. J. and Hall, A., Cell 70:389-399 (1992); Leung, T. et al. Mol. Cell Biol. 16:5313-5327(1996); Amano, M. et al. Science 275:1308-1311 (1997)). Although RHO activity is important for cellular motility, efficient migration requires a tightly balanced activation and deactivation of RAC1, CDC42, and RhoA at both appropriate space and time in the cellular environment. RHO proteins also participate in the control of gene transcription, cell cycle progression, or anti-apoptotic pathways (Etienne-Manneville, S. and Hall, A., Nature 420:629-635 (2002)), and recent study has shown that RhoA is activated in some human tumors (Sahai, E. and Marshall, C., J., Nat. Rev. Cancer 2(2):133-142 (2002)), although its precise mechanism especially upstream pathway of RhoA signaling during carcinogenesis has not been clarified.

### SUMMARY OF THE INVENTION

The present invention is based on the finding that ANLN and RhoA interact in non-small cell lung cancer cells (NSCLC) and, further, that nuclear localization of ANLN is associated with poor prognosis in non-small cell lung cancer patients. Accordingly, novel methods for identifying compounds that slow or arrest the progression of cancer, *e*.*g*., non-small cell lung cancer are described, by interfering with ANLN/RhoA interaction or by inhibiting ANLN-mediated cell motility. The invention provides methods for determining a prognosis for NSCLC cancer patients by determining the presence or absence of nuclear localization of ANLN in a sample.

Accordingly, methods of screening for compounds useful in treating or preventing cancer, particularly a lung cancer such as non-small cell lung cancer (NSCLC) are described. In some embodiments, the methods comprise the steps of:
(a) contacting a polypeptide comprising an RhoA-binding domain of an ANLN polypeptide with a polypeptide comprising an ANLN-binding domain of an RhoA polypeptide in the presence of a test compound;
(b) detecting binding between the polypeptides; and
(c) selecting the test compound that inhibits binding between the polypeptides.

In some embodiments, the polypeptide comprising the RhoA-binding domain comprises an ANLN polypeptide. Similarly, in other embodiments, the polypeptide comprising the ANLN-binding domain comprises an RhoA polypeptide.

In some embodiments, the polypeptide comprising the RhoA-binding domain is expressed in a living cell.

In some embodiments, the binding between the polypeptides is detected by a method comprising the step of detecting:
(a) the concentration of activated RhoA;
(b) the interaction between RhoA and an RHO effector or an RhoA-binding region thereof;
(c) the activation of any signal complex, including downstream gene expression or downstream gene product activity mediated by activated RhoA;
(d) the promotion of DNA synthesis and cell cycle entry;
(e) cell migration or any other oncogenic phenotype;
(f) actin stress fiber formation and F-actin production; and
(g) the interaction with any molecules important for cell adhesion, migration and invasion.

The present invention also describes kits for screening for a compound useful in treating or preventing cancer, particularly a lung cancer such as non-small cell lung cancer (NSCLC). In some embodiments, the kit comprises:
(a) a first polypeptide comprising an RhoA-binding domain of an ANLN polypeptide;
(b) a second polypeptide comprising an ANLN-binding domain of an RhoA polypeptide, and
(c) means (*e.g*., a reagent) to detect the interaction between the first and second polypeptides.

In some embodiments, the first polypeptide, *i*.*e*., the polypeptide comprising the RhoA-binding domain, comprises an ANLN polypeptide. Similarly, in other embodiments, the second polypeptide, *i*.*e*., the polypeptide comprising the ANLN-binding domain, comprises an RhoA polypeptide.

In some embodiments, the polypeptide comprising the RhoA-binding domain is expressed in a living cell.

In some embodiments, the means (*e.g*., the reagent) to detect the interaction between the two polypeptides detects:
(a) the concentration of activated RhoA;
(b) the interaction between RhoA and an RHO effector or an RhoA-binding region thereof;
(c) the activation of any signal complex, including downstream genes mediated by activated RhoA;
(d) the promotion of DNA synthesis and cell cycle entry;
(e) cell migration or any other oncogenic phenotype;
(f) actin stress fiber formation and F-actin production; and
(g) the interaction with any molecules important for cell adhesion, migration and invasion.

In an alternate embodiment, the method of screening for a compound useful in treating or preventing cancer, particularly a lung cancer such as non-small cell lung cancer (NSCLC), of the present invention comprises the steps of:
(a) contacting a cell expressing an ANLN polypeptide, or a functional equivalent thereof, with a test compound;
(b) detecting ANLN-mediated motility of the cell; and
(c) selecting the test compound that inhibits the motility of the cell, as compared to a motility level detected in the absence of the test compound.

In some embodiments, the cell comprises a vector, the vector comprising a polynucleotide encoding an ANLN polypeptide, or a functional equivalent thereof, in an expressible manner (*e.g*., as a promoter operably linked to a polynucleotide encoding the ANLN polypeptide).

Similarly, a kit for screening for a compound useful in treating or preventing cancer, particularly a lung cancer such as non-small cell lung cancer (NSCLC), is described and may comprise:
(a) a cell expressing an ANLN polypeptide or a functional equivalent thereof; and
(b) means (*e.g*., a reagent) to detect the motility of the cell.

The present invention provides methods of predicting a cancer prognosis in a patient afflicted with non-small cell lung cancer (NSCLC), the method comprises the steps of:
(a) detecting an ANLN localized in the nucleus of a specimen collected from a subject whose NSCLC prognosis is to be predicted, and
(b) predicting a poor prognosis when localization of the ANLN in the nucleus is detected.

In some embodiments, the localization of ANLN in the nucleus of the specimen is detected by:
(a) contacting an antibody recognizing the ANLN protein with the specimen; and
(b) detecting the antibody which binds to the specimen in the nuclear region.

The present invention also describes kits for predicting a prognosis of a cancer in a subject, particularly the prognosis of a lung cancer such as non-small cell lung cancer (NSCLC). In some embodiments, the kits comprise (a) an antibody recognizing an ANLN protein and (b) an agent for detection in the nucleus. In some embodiments, the agent for detection of the nucleus is hematoxylin-eosin staining dye.

The present invention also describes methods for treating or preventing cancer in a subject, particularly a lung cancer such as non-small cell lung cancer (NSCLC). In some embodiments, the method comprises the step of administering a compound selected by the steps of:
(a) contacting a polypeptide comprising an RhoA-binding domain of an ANLN polypeptide with a polypeptide comprising an ANLN-binding domain of an RhoA polypeptide in the presence of a test compound;
(b) detecting binding between the polypeptides; and
(c) selecting the test compound that inhibits the binding between the polypeptides.

Alternatively, the method may comprise the steps of:
(a) contacting a cell expressing an ANLN polypeptide, or a functional equivalent thereof, with a test compound;
(b) detecting the motility of the cell; and
(c) selecting the test compound that inhibits the motility of the cell, as compared to a motility level detected in the absence of the test compound.

The present invention also describes methods for treating or preventing cancer in a subject, particularly a lung cancer such as non-small cell lung cancer (NSCLC), wherein the method comprises the step of administering a compound that inhibits binding between ANLN and RhoA.

The present invention also describes compositions useful in treating or preventing cancer, particularly a lung cancer such as non-small cell lung cancer (NSCLC), wherein the composition comprises a pharmaceutically effective amount of the compound selected by the steps of:
(a) contacting a polypeptide comprising an RhoA-binding domain of an ANLN polypeptide with a polypeptide comprising an ANLN-binding domain of an RhoA polypeptide in the presence of a test compound;
(b) detecting binding between the polypeptides; and
(c) selecting the test compound that inhibits the binding between the polypeptides;
or, alternatively, selected by the steps of:
(a) contacting a cell expressing an ANLN polypeptide, or functional equivalent thereof, with a test compound;
(b) detecting the motility of the cell; and
(c) selecting the test compound that inhibits the motility of the cell, as compared to a motility level detected in the absence of the test compound.

The present invention also describes compositions useful in treating or preventing cancer, particularly a lung cancer such as non-small cell lung cancer (NSCLC), wherein the composition comprises a pharmaceutically effective amount of a compound that inhibits binding between the ANLN and RhoA, and a pharmaceutically acceptable carrier.

### BRIEF DESCRIPTION OF THE DRAWINGS

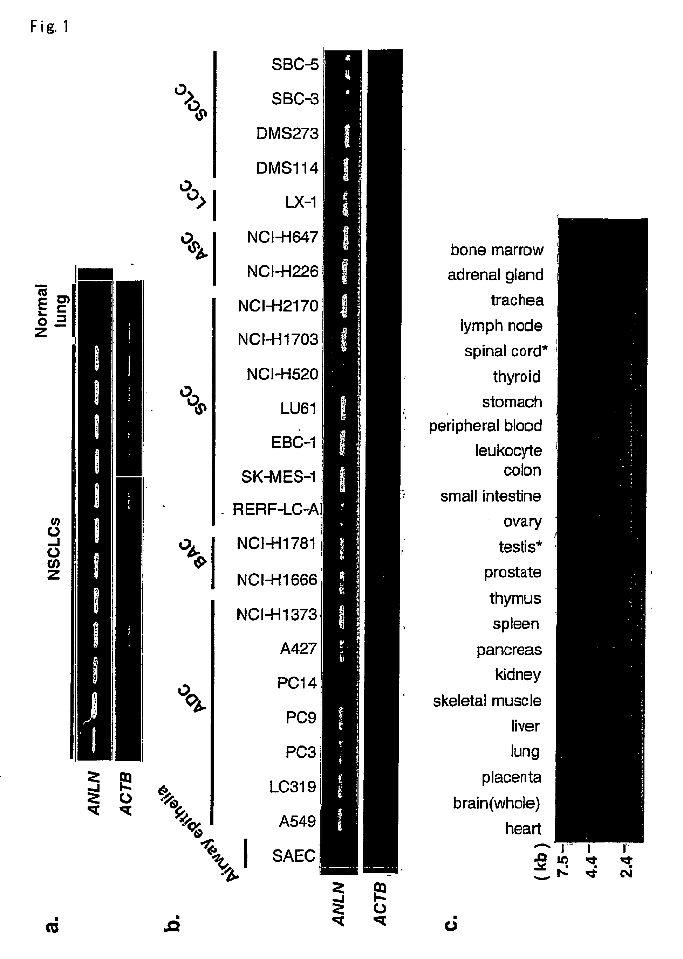

Figure 1 illustrates the *ANLN* expression in primary NSCLCs and lung-cancer cell lines. Part (a) depicts the expression of *ANLN* in clinical samples of NSCLC and normal lung tissues, examined by semi-quantitative RT-PCR. Part (b) depicts the expression of *ANLN* in lung-cancer cell lines. Part (c) depicts the expression of *ANLN* in normal tissues as detected by Northern-blot analysis.

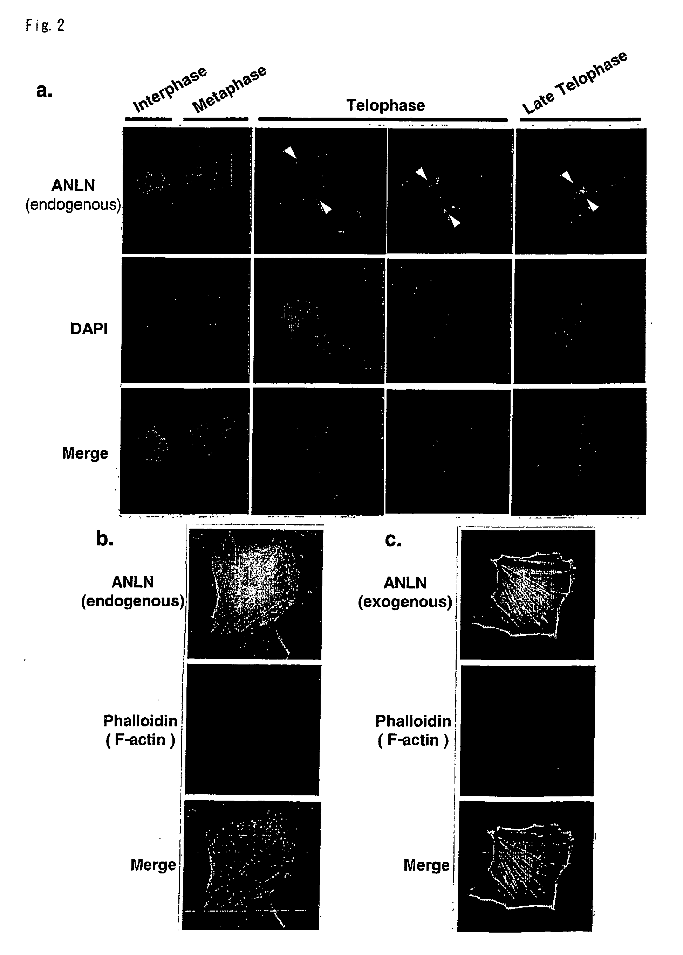

Figure 2 illustrates the subcellular localization of ANLN and actin stress fiber formation. Part (a) depicts the subcellular localization of endogenous ANLN in the NSCLC cell line, LC319. ANLN expression in the nucleus (n-ANLN), cytoplasm (c-ANLN), and cleavage furrow was detected by immunocytochemical staining using rhodamine-conjugated secondary antibody. Part (b) depicts the co-localization of endogenous c-ANLN and F-actin in LC319 cells detected with FITC-conjugated secondary antibody and Alexa594-conjugated phalloidin, respectively. In part (c), LC319 cells were transiently transfected with ANLN-expressing plasmids 24 hours before analysis, and ANLN and F-actin distribution were assessed by FITC-immunostaining for ANLN and Alexa594-phalloidin staining. Induction of stress fiber formation by exogenous ANLN expression and their co-localization were observed in LC319 cells.

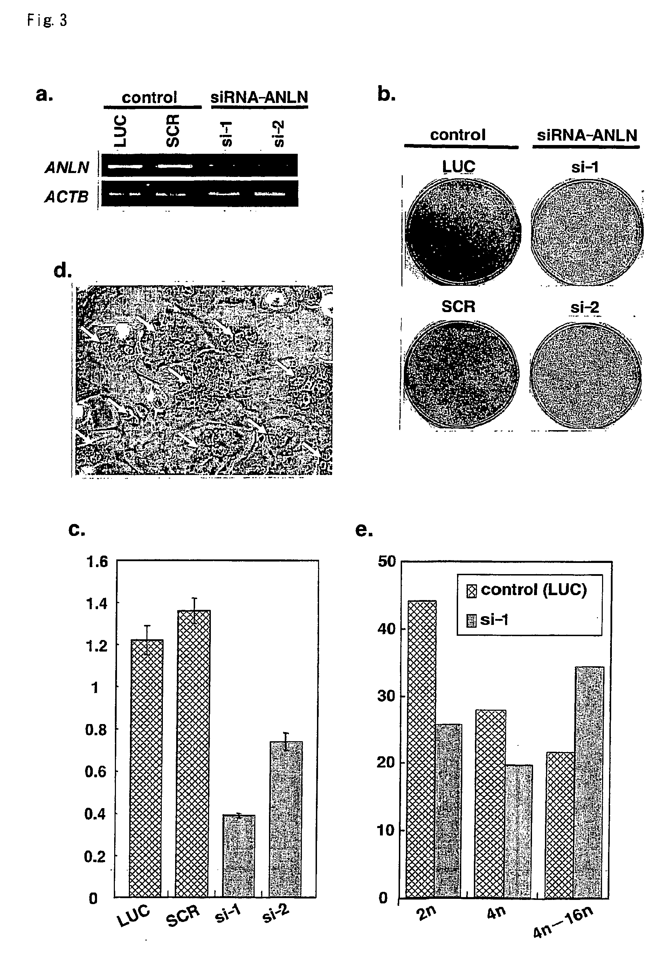

Figure 3 illustrates the inhibition of growth of NSCLC cells by siRNA against *ANLN.* Part (a) depicts the expression of *ANLN* in response to siRNA-*ANLN*-1 (ski-1), -2 (si-2) or control siRNAs against luciferase (LUC) or scramble (SCR) in LC319 cells, analyzed by semi-quantitative RT-PCR. Part (b) depicts colony-formation assays of LC319 cells transfected with specific siRNAs for *ANLN* (si-1 and -2) or control plasmids (si-LUC and - SCR). Part (c) depicts the viability of LC319 cells evaluated by MTT assay in response to si-1, -2, -LUC, or -SCR. Part (d) depicts the microscopic observation of LC319 cells transfected with si-1 or -LUC. Arrow indicates the cells treated with siRNA-*ANLN*-1 (si-1) showing multinucleated and larger cell morphology. Part (e) depicts the results of flow cytometric analysis of LC319 cells transfected with si-1 or -LUC. The proportion of cells with a DNA content of 4N-16N in the cells transfected with si-1 was significantly higher than that in the cells transfected with control siRNA (si-LUC). Assays were performed three times, and in triplicate wells.

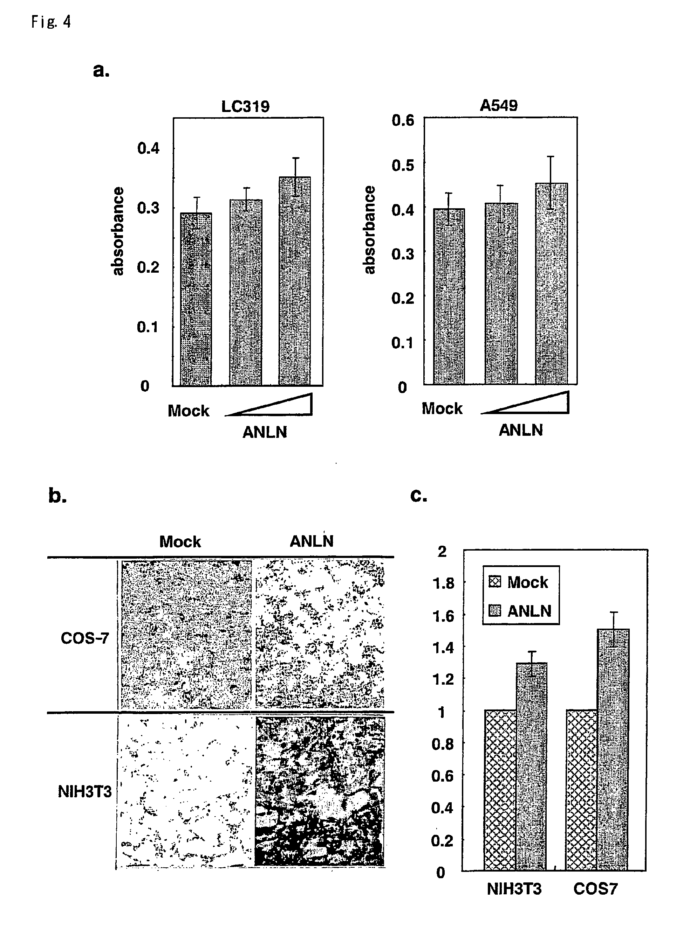

Figure 4 illustrates the promotion of DNA synthesis and activation of cellular motility by ANLN. Part (a) depicts the BrdU incorporation in LC319 or A549 cells transiently transfected with ANLN after 20 hours incubation with BrdU. DNA synthesis was likely to be promoted in a dose dependent manner in both LC319 and A549 cells transiently transfected with ANLN-expressing plasmids. Parts (b) and (c) depict the results of a Matrigel invasion assay demonstrating the promotion ofNIH3T3 and COS-7 cell invasive nature in Matrigel matrix when the human ANLN expression plasmids were transfected. Specifically, part (b) shows Giemsa staining (X100) and part (c) represents number of cells migrating through the Matrigel-coated filters. Assays were performed three times, and in triplicate wells.

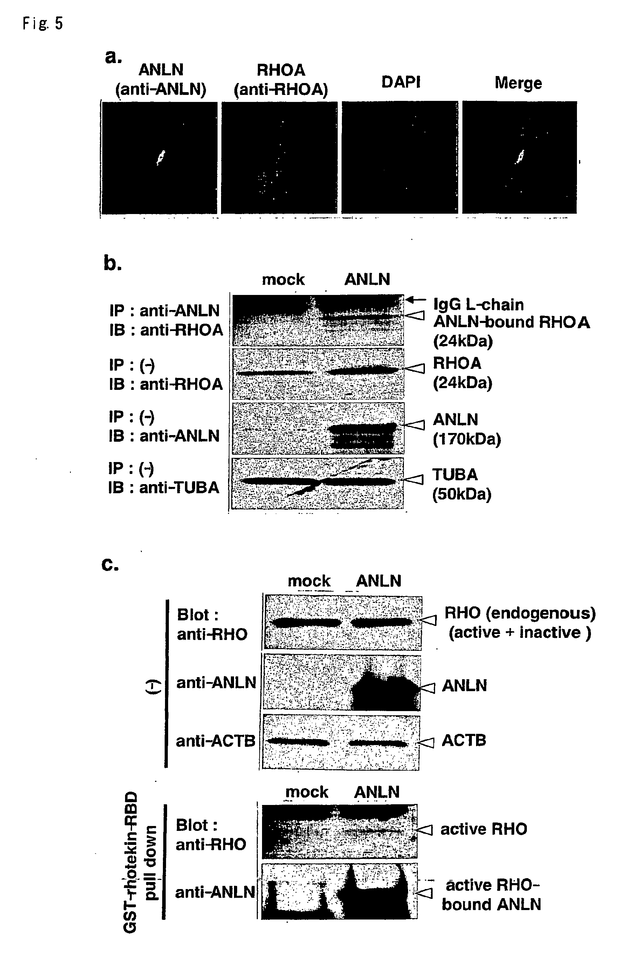

Figure 5 illustrates the interaction of ANLN with RhoA and its regulation of RHO activation. Part (a) depicts the co-localization of endogenous ANLN and RhoA was detected by immunocytochemical staining using anti-ANLN antibody (FITC) and anti-RhoA antibody (rhodamine). Part (b) depicts the immunoprecipitation (IP) of exogenous ANLN and endogenous RhoA from lung-cancer cell line LC319 extracts. LC319 cells were transfected with mock or ANLN and were subjected to ANLN-IP with anti-ANLN antibody followed by immunoblotting (IB) with anti-RhoA antibody (*top*). Aliquots of cell lysates were directly subjected to immunoblotting to confirm the expression of each protein as indicated in *lower three panels*. Part (c) depicts RHO activation induced by direct binding of ANLN. (*upper three panels*) LC319 cells were transfected with mock or ANLN and were directly subjected to immunoblotting to confirm the expression of each protein. (*lower two panels*) Aliquots of cell lysates were incubated with GST-RTKN-RBD and subjected to GST pull-down assay, and were then subjected to immunoblotting with anti-RHO and anti-ANLN antibody. The *first panel* shows the level of RHO activated by exogenous ANLN expression, and the *second panel* demonstrates the direct interaction of ANLN with activated RHO.

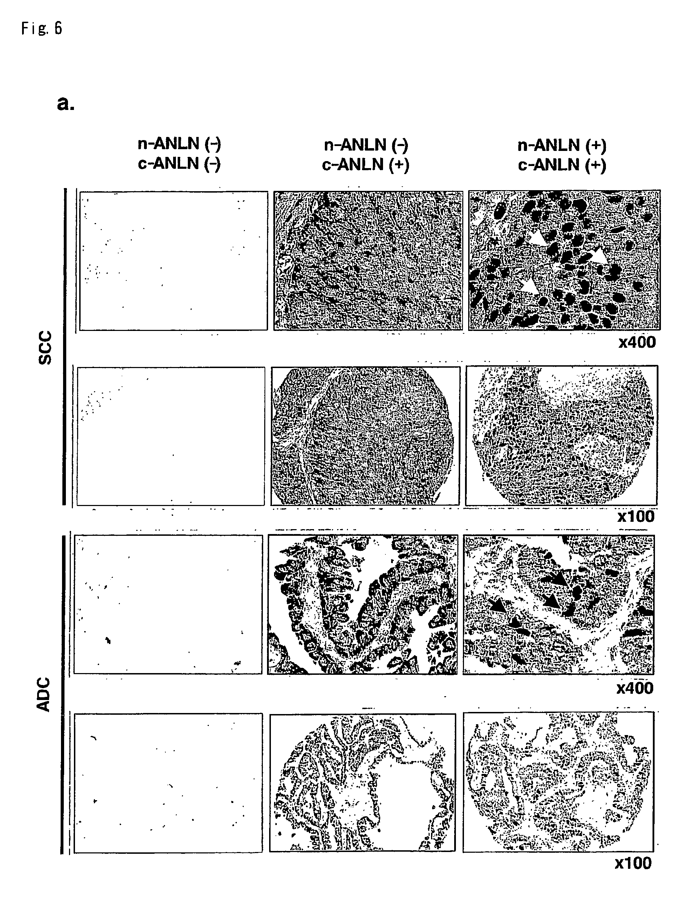

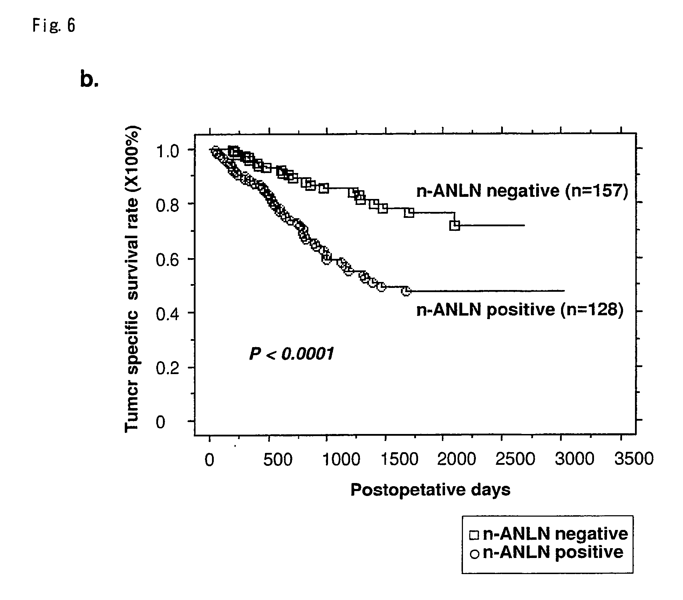

Figure 6 illustrates that the over-expression of n-ANLN is associated with a worse outcome in NSCLC. Part (a) depicts the results of immunohistochemical evaluation of representative samples from surgically-resected NSCLC tissues using anti-ANLN polyclonal antibody on tissue microarrays (ADC, SCC, X100). c-ANLN indicates ANLN that localized at cytoplasm; n-ANLN, at nuclei. Arrow indicates examples of the cells expressing n-ANLN. Part (b) depicts the results of tissue microarray and Kaplan-Meier analysis of tumor specific survival in patients with NSCLC according to n-ANLN expression (P < 0.0001; Log-rank test).

### DETAILED DESCRIPTION OF THE PREFERRED EMBODIMENTS

### I. Overview

Molecular-targeted drugs are expected to be highly specific to malignant cells, with minimal adverse effects due to their well-defined mechanisms of action. As a promising strategy to identify appropriate molecular targets for development of such drugs, the present inventors combined the genome-wide expression analysis that could select genes over-expressed in cancer cells, with high-throughput screening of loss-of-function effects by means of the RNAi technique. In addition, the tissue microarray method was applied to analyze hundreds of archived clinical samples for validation of the potential target proteins. Using this kind of the systematic approach, it is demonstrated herein that ANLN is frequently over-expressed in clinical NSCLC samples as well as cell lines, and that this gene product plays indispensable roles in the growth and progression of lung-cancer cells.

ANLN was initially characterized as a human homologue of anillin, a *Drosophila* actin binding protein (Oegema, K.et al. J. Cell Biol. 150(3):539-551 (2000)). The human ANLN cDNA encodes a 1124-amino-acid protein including the actin-binding domain and C-terminal pleckstrin homology (PH) domain. It also contained several consensus nuclear localization sequences (NLS) and one consensus SH3-binding motif. ANLN localizes to the cleavage furrow during cytokinesis and is supposed to play an important role in cytokinesis. Herein, it was discovered that ANLN localized not only to the cytoplasm but also to the nuclei in some proportion of cancer cells, while, it located at the cortex following nuclear envelope breakdown, and the cleavage furrow during cytokinesis. As reported previously, ANLN is likely to play an important role in cell-cycle progression and in the late phases may assemble the actin and myosin contractile ring that separates daughter cells through interaction with at least two other furrow proteins, actin and septins (SEPTs) (Oegema, K.et al. J. Cell Biol. 150(3):539-551 (2000)). As discussed herein, it has been observed that NSCLC cells treated with *ANLN*-siRNA showed furrow regression and larger cell morphology, and became multinucleated probably due to dysfunction of cytokinesis process as a consequence of prevention of the assembly of the contractile ring. Herein, it was discovered that the interaction of endogenous RhoA (SEQ ID NO:4, encoded by SEQ ID NO:3) with ANLN occurred not only in cytoplasm, but also in the cleavage furrow and the midbody, indicating that cell growth was promoted through ANLN-RhoA interaction and acceleration of cytokinesis of NSCLC cells.

The present inventors then focused on the effect of ANLN on RHO activation, which is known to control the formation of actin structures (Ridley, A. J. and Hall, A. Cell 70:389-399 (1992)), and found that over-expression of exogenous ANLN promoted the formation of actin stress fibers in mammalian cells. The data herein suggest that ANLN activates the RHO signaling through its interaction with RhoA, resulting in significant promotion of reorganization of the actin cytoskeleton and might consequently activate cellular migration activity, as observed by Matrigel invasion and Wound migration assays.

RAS and RHO GTPases are well-studied signaling molecules; the RHO GTPases have emerged as key molecules in regulating a diverse set of biological activities including actin organization, focal complex/adhesion assembly, cell motility, cell polarity, gene transcription and cell-cycle progression. Recent works have shown that RHO proteins are over-expressed in several human tumors and that some growth factors including epidermal growth factor (EGF), hepatocyte growth factor (HGF), lypophosphatidic acid (LPA), platelet-derived growth factor (PDGF) and transforming growth factor-beta (TGFB) could activate RHO proteins (Zondag, G. C. et al. J. Cell Biol. 149:775-781 (2000); Bhowmick, N. A., et al., Mol. Biol. Cell 12, 27-36 (2001); Liu, A. X.et al., Mol. Cell Biol. 21:6906-6912 (2001)). Several classes of cell adhesion molecules including integrins, cadherins, and immunoglobulin superfamily members have also been shown to affect RHO activities (DeMali, K. A., et al., Curr. Opin. Cell Biol. 15:572-582 (2003); Braga, V.M., Curr. Opin. Cell Biol. 14, 546-556 (2002); Thompson, P. W., et al., J. Immunol. 169:1007-1013 (2002)). In addition, several guanine nucleotide exchange factors (GEFs) may abnormally activate RHO proteins and their downstream effectors, resulting in neoplastic transformation (Fort, P., Prog. Mol. Subcell. Biol. 22, 159-181 (1999); Zohn, I. M., et al., Oncogene 17, 1415-1438 (1998)). Our data have suggested that aberrant activation of the RhoA by over-expressed ANLN promoted migration activity of the mammalian cells through reorganization of the actin cytoskeleton and could contribute to invasive and metastatic potential of cancer cells.

BrdU incorporation assay detected the promotion of DNA synthesis of the NSCLC cells by exogenously expressed ANLN in a dose dependent manner, suggesting that ANLN could be an important positive regulator of cell cycle progression. RHO GTPases such as RHO, RAC and CDC42 are known to contribute different activities to the G1 phase of the cell cycle (Olson, M. F., et al., Science 269, 1270-1272 (1995)). *In vitro* studies have revealed a multiple mechanisms by which RHO proteins can promote cell cycle progression mainly mediated by the regulation of cyclin-dependent kinases (CDKs), whose activities are controlled by the activators, such as cyclin D1 (CCND1), and inhibitors, such as the CDK inhibitors (CDKIs) p21^{WAF1} and p27^{KIP1} (Danen, E. H., et al., J. Cell Biol. 151, 1413-1422 (2000); Olson, M. F., et al., Nature 394:295-299 (1998); Adnane, J., et al., Mol. Cell. Biol. 18:6962-6970 (1998)). The promotion of DNA synthesis of the NSCLC cells by ANLN may be due to the up-regulation of activated RhoA that affects the function of these molecular pathways.

The results of the tissue microarray experiments presented herein demonstrate that lung-cancer patients with n-ANLN positive tumors showed a poor cancer-specific survival compared with those without the negative tumors. Although the precise molecular mechanism of the ANLN transport to the nucleus and whether it has nucleus-specific additional function is not clear, the data suggest that n-ANLN contributes to the very malignant phenotype of lung-cancer cells by activating some unidentified signaling pathway(s).

In summary, ANLN is demonstrated herein to directly interact with and activate RhoA, and this complex is likely to be essential for growth-promoting pathway and aggressive features of lung cancers as well as cell division/cell cycle progression. The data reported here demonstrate that this ANLN-RhoA pathway can be a good molecular target to design a novel biomarker and develop anti-cancer drugs specific to lung cancer. Therapeutic siRNAs should be one of the options to interfere with this pathway.

### II. Definitions:

The words "a", "an", and "the" as used herein mean "at least one" unless otherwise specifically indicated.

In the context of the present invention, an "ANLN polypeptide" or "ANLN" refers to an actin binding protein often referred to as "anillin" in the scientific literature. *See, e.g.,* Oegema et al., J. Cell Biol. 150(3):539-551 (2000). ANLN polypeptides may be substantially identical to the human (Genbank accession No. AF273437), *Drosophila* (Genbank accession No. X89858, the product of the *Drosophila* gene CG4530 (GenBank accession No. AAF47044)), the products of the *C. elegans* genes K10B2.5 (GenBank accession No. T16604), Y43F8C.14 (GenBank accession No. T26874), and Y49E10.19 (GenBank accession No. T27053), or other orthologous polypeptides. In some embodiments, the ANLN polypeptides comprise the amino acids conserved between the human and *Drosophila* ANLN orthologs. Alternatively, the ANLN polypeptides may comprise amino acids conserved between all of the above-listed proteins, *e*.*g*., as displayed on page 542 of Oegema *et al.* Full length ANLN polypeptides may comprise, *e*.*g*., a PH binding domain, an actin binding domain, nuclear localization signal sequences, and SH3 domain(s).

Herein, an "RhoA polypeptide" or "RhoA" refers to the Rho gene family of GTP binding proteins. The crystal structure of RhoA has been determined. *See, e.g.,* Maesaki et al., Molec. Cell 4:793-803 (1999). Maesaki *et al.* provide a significant amount of information regarding the primary and secondary structure of RhoA, including the location and structure of an ACC finger structure that distinguishes RhoA from other members of the Rho family. Examples of RhoA polypeptides include, *e.g*., proteins substantially identical to SEQ ID NO:4.

Recent studies have shown the RHO family of proteins to be involved in tumorigenesis. Though the pathways remain unclear, the links between RHO and cancer are substantial. The RhoA proteins in particular seem to have extensive links to cancer, with the RhoA over-expression being linked to colon, breast, lung, testicular germ cell and head and neck squamous cell carcinoma tumors. In addition, it has been suggested that RhoA is involved in cell motility and cell polarity. The effect of RhoA expression on these two functions suggest a likely cause for the formation of tumors.

In the context of the present invention, "inhibition of binding" between two proteins refers to at least reducing binding between the proteins. Thus, in some cases, the percentage of binding pairs in a sample will be decreased compared to an appropriate (*e.g*., not treated with test compound or from a non-cancer sample, or from a cancer sample) control. The reduction in the amount of proteins bound may be, *e.g*., less than 90%, 80%, 70%, 60%, 50%, 40%, 25%, 10%, 5%, 1% or less (*e.g*., 0%), than the pairs bound in a control sample.

The term "test compound" refers to any (*e.g*., chemically or recombinantly-produced) molecule that may disrupt the protein-protein interaction between ANLN and RhoA, as discussed in detail herein. In some embodiments, the test compounds have a molecular weight of less than 1,500 daltons, and in some cases less than 1,000, 800, 600, 500, or 400 daltons.

A "pharmaceutically effective amount" of a compound is a quantity that is sufficient to treat and/or ameliorate an ANLN-mediated disease in an individual. An example of a pharmaceutically effective amount may an amount needed to decrease the interaction between ANLN and RhoA when administered to an animal. The decrease in interaction may be, *e.g*., at least a 5%, 10%, 20%, 30%, 40%, 50%, 75%, 80%, 90%, 95%, 99%, or 100% change in binding. Alternatively, the amount may comprise an amount that, when administered, results in detectably decreased nuclear localization as described herein for wild-type ANLN.

The phrase "pharmaceutically acceptable carrier" refers to an inert substance used as a diluent or vehicle for a drug.

In the context of the present invention, the term "functionally equivalent" means that the subject polypeptide has a biological activity of a reference polypeptide. For example, a functional equivalent of ANLN would have the ability to bind actin *in vitro* and induce cellular motility like wild-type ANLN. Assays for determining such activity are well known in the art.

The terms "isolated" and "biologically pure" refer to material that is substantially or essentially free from components which normally accompany it as found in its native state. However, the term "isolated" is not intended to refer to the components present in an electrophoretic gel or other separation medium. An isolated component is free from such separation media and in a form ready for use in another application or already in use in the new application/milieu.

The phrase "conservatively modified variants" applies to both amino acid and nucleic acid sequences. With respect to particular nucleic acid sequences, conservatively modified variants refers to those nucleic acids which encode identical or essentially identical amino acid sequences, or where the nucleic acid does not encode an amino acid sequence, to essentially identical sequences. Because of the degeneracy of the genetic code, a large number of functionally identical nucleic acids encode any given protein. For instance, the codons GCA, GCC, GCG and GCU all encode the amino acid alanine. Thus, at every position where an alanine is specified by a codon, the codon can be altered to any of the corresponding codons described without altering the encoded polypeptide. Such nucleic acid variations are "silent variations," which are one species of conservatively modified variations. Every nucleic acid sequence herein which encodes a polypeptide also describes every possible silent variation of the nucleic acid. One of skill will recognize that each codon in a nucleic acid (except AUG, which is ordinarily the only codon for methionine, and TGG, which is ordinarily the only codon for tryptophan) can be modified to yield a functionally identical molecule. Accordingly, each silent variation of a nucleic acid that encodes a polypeptide is implicitly described in each disclosed sequence.

As to amino acid sequences, one of skill will recognize that individual substitutions, deletions or additions to a nucleic acid, peptide, polypeptide, or protein sequence which alters, adds or deletes a single amino acid or a small percentage of amino acids in the encoded sequence is a "conservatively modified variant" wherein the alteration results in the substitution of an amino acid with a chemically similar amino acid. Conservative substitution tables providing functionally similar amino acids are well known in the art. Such conservatively modified variants are in addition to and do not exclude polymorphic variants, interspecies homologs, and alleles of the invention.

The following eight groups each contain amino acids that are conservative substitutions for one another:
1) Alanine (A), Glycine (G);
2) Aspartic acid (D), Glutamic acid (E);
3) Asparagine (N), Glutamine (Q);
4) Arginine (R), Lysine (K);
5) Isoleucine (I), Leucine (L), Methionine (M), Valine (V);
6) Phenylalanine (F), Tyrosine (Y), Tryptophan (W);
7) Serine (S), Threonine (T); and
8) Cysteine (C), Methionine (M) (*see, e*.*g.,* Creighton, Proteins (1984)).

In the context of the present invention, a "percentage of sequence identity" is determined by comparing two optimally aligned sequences over a comparison window, wherein the portion of the polynucleotide sequence in the comparison window may comprise additions or deletions (*i*.*e*., gaps) as compared to the reference sequence (*e.g*., a polypeptide of the invention), which does not comprise additions or deletions, for optimal alignment of the two sequences. The percentage is calculated by determining the number of positions at which the identical nucleic acid base or amino acid residue occurs in both sequences to yield the number of matched positions, dividing the number of matched positions by the total number of positions in the window of comparison and multiplying the result by 100 to yield the percentage of sequence identity.

The terms "identical" or percent "identity," in the context of two or more nucleic acids or polypeptide sequences, refer to two or more sequences or subsequences that are the same sequences. Two sequences are "substantially identical" if two sequences have a specified percentage of amino acid residues or nucleotides that are the same (*i*.*e*., 60% identity, optionally 65%, 70%, 75%, 80%, 85%, 90%, or 95% identity over a specified region, or, when not specified, over the entire sequence), when compared and aligned for maximum correspondence over a comparison window, or designated region as measured using one of the following sequence comparison algorithms or by manual alignment and visual inspection. Optionally, the identity exists over a region that is at least about 50 nucleotides in length, or more preferably over a region that is 100 to 500 or 1000 or more nucleotides in length.

For sequence comparison, typically one sequence acts as a reference sequence, to which test sequences are compared. When using a sequence comparison algorithm, test and reference sequences are entered into a computer, subsequence coordinates are designated, if necessary, and sequence algorithm program parameters are designated. Default program parameters can be used, or alternative parameters can be designated. The sequence comparison algorithm then calculates the percent sequence identities for the test sequences relative to the reference sequence, based on the program parameters.

A "comparison window", as used herein, includes reference to a segment of any one of the number of contiguous positions selected from the group consisting of from 20 to 600, usually about 50 to about 200, more usually about 100 to about 150 in which a sequence may be compared to a reference sequence of the same number of contiguous positions after the two sequences are optimally aligned. Methods of alignment of sequences for comparison are well known in the art. Optimal alignment of sequences for comparison can be conducted, *e.g*., by the local homology algorithm of Smith and Waterman (1981) Adv. Appl. Math. 2:482-489, by the homology alignment algorithm of Needleman and Wunsch (1970) J. Mol. Biol. 48:443, by the search for similarity method of Pearson and Lipman (1988) Proc. Nat'l. Acad. Sci. USA 85:2444, by computerized implementations of these algorithms (GAP, BESTFIT, FASTA, and TFASTA in the Wisconsin Genetics Software Package, Genetics Computer Group, 575 Science Dr., Madison, WI), or by manual alignment and visual inspection (*see, e*.*g*., Ausubel et al., Current Protocols in Molecular Biology (1995 supplement)).

Two examples of algorithms that are suitable for determining percent sequence identity and sequence similarity are the BLAST and BLAST 2.0 algorithms, which are described in Altschul et al. (1997) Nuc. Acids Res. 25:3389-3402, and Altschul et al. (1990) J. Mol. Biol. 215:403-410, respectively. Software for performing BLAST analyses is publicly available through the National Center for Biotechnology Information. This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Altschul *et al., supra*). These initial neighborhood word hits act as seeds for initiating searches to find longer HSPs containing them. The word hits are extended in both directions along each sequence for as far as the cumulative alignment score can be increased. Cumulative scores are calculated using, for nucleotide sequences, the parameters M (reward score for a pair of matching residues; always > 0) and N (penalty score for mismatching residues; always < 0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLASTN program (for nucleotide sequences) uses as defaults a word length (W) of 11, an expectation (E) of 10, M=5, N=-4 and a comparison of both strands. For amino acid sequences, the BLASTP program uses as defaults a word length of 3, and expectation (E) of 10, and the BLOSUM62 scoring matrix (*see* Henikoff and Henikoff (1989) Proc. Natl. Acad. Sci. USA 89:10915) alignments (B) of 50, expectation (E) of 10, M=5, N=-4, and a comparison of both strands.

The BLAST algorithm also performs a statistical analysis of the similarity between two sequences (*see, e.g.,* Karlin and Altschul (1993) Proc. Natl. Acad. Sci. USA 90:5873-7). One measure of similarity provided by the BLAST algorithm is the smallest sum probability (P(N)), which provides an indication of the probability by which a match between two nucleotide or amino acid sequences would occur by chance. For example, a nucleic acid is considered similar to a reference sequence if the smallest sum probability in a comparison of the test nucleic acid to the reference nucleic acid is less than about 0.2, more preferably less than about 0.01, and most preferably less than about 0.001.

The term "small organic molecules" refers to molecules of a size comparable to those organic molecules generally used in pharmaceuticals. The term excludes biological macromolecules (*e.g*., proteins, nucleic acids, *etc*.). Preferred small organic molecules range in size up to about 5000 Da, *e.g*., up to 2000 Da, or up to about 1000 Da.

The terms "label" and "detectable label" are used herein to refer to any composition detectable by spectroscopic, photochemical, biochemical, immunochemical, electrical, optical or chemical means. Such labels include biotin for staining with labeled streptavidin conjugate, magnetic beads (*e.g*., DYNABEADS™), fluorescent dyes (*e.g*., fluorescein, Texas red, rhodamine, green fluorescent protein, and the like), radiolabels (*e*.*g*., ³H, ¹²⁵I, ³⁵S, ¹⁴C, or ³²P), enzymes (*e.g*., horse radish peroxidase, alkaline phosphatase and others commonly used in an ELISA), and calorimetric labels such as colloidal gold or colored glass or plastic (*e.g*., polystyrene, polypropylene, latex, *etc*.) beads. Patents teaching the use of such labels include U.S. Pat. Nos. 3,817,837; 3,850,752; 3,939,350; 3,996,345; 4,277,437; 4,275,149; and 4,366,241. Means of detecting such labels are well known to those of skill in the art. Thus, for example, radiolabels may be detected using photographic film or scintillation counters, fluorescent markers may be detected using a photodetector to detect emitted light. Enzymatic labels are typically detected by providing the enzyme with a substrate and detecting, the reaction product produced by the action of the enzyme on the substrate, and calorimetric labels are detected by simply visualizing the colored label.

The term "antibody" as used herein encompasses naturally occurring antibodies as well as non-naturally occurring antibodies, including, for example, single chain antibodies, chimeric, bifunctional and humanized antibodies, as well as antigen-binding fragments thereof, (*e*.*g*., Fab', F(ab')₂, Fab, Fv and rIgG). *See also*, Pierce Catalog and Handbook, 1994-1995 (Pierce Chemical Co., Rockford, IL). *See also, e*.*g*., Kuby, J., Immunology, 3rd Ed., W.H. Freeman & Co., New York (1998). Such non-naturally occurring antibodies can be constructed using solid phase peptide synthesis, can be produced recombinantly or can be obtained, for example, by screening combinatorial libraries consisting of variable heavy chains and variable light chains as described by Huse et al., Science 246:1275-1281 (1989), which is incorporated herein by reference. These and other methods of making, for example, chimeric, humanized, CDR-grafted, single chain, and bifunctional antibodies are well known to those skilled in the art (Winter and Harris, Immunol. Today 14:243-246 (1993); Ward et al., Nature 341:544-546 (1989); Harlow and Lane, Antibodies, A Laboratory Manual, 1988; Hilyard et al., Protein Engineering: A practical approach (IRL Press 1992); Borrebeck, Antibody Engineering, 2d ed. (Oxford University Press 1995).

The term "antibody" includes both polyclonal and monoclonal antibodies. The term also includes genetically engineered forms such as chimeric antibodies (*e.g*., humanized murine antibodies) and heteroconjugate antibodies (*e.g*., bispecific antibodies). The term also refers to recombinant single chain Fv fragments (scFv). The term antibody also includes bivalent or bispecific molecules, diabodies, triabodies, and tetrabodies. Bivalent and bispecific molecules are described in, *e.g*., Kostelny et al. (1992) J Immunol 148:1547, Pack and Pluckthun (1992) Biochemistry 31:1579, Holliger et al. (1993) Proc Natl Acad Sci U S A. 90:6444, Gruber et al. (1994) J Immunol 152:5368, Zhu et al. (1997) Protein Sci 6:781, Hu et al. (1996) Cancer Res. 56:3055, Adams et al. (1993) Cancer Res. 53:4026, and McCartney, et al. (1995) Protein Eng. 8:301.

Typically, an antibody has a heavy and light chain. Each heavy and light chain contains a constant region and a variable region, (the regions are also known as "domains"). Light and heavy chain variable regions contain four "framework" regions interrupted by three hypervariable regions, also called "complementarity-determining regions" or "CDRs". The extent of the framework regions and CDRs have been defined. The sequences of the framework regions of different light or heavy chains are relatively conserved within a species. The framework region of an antibody, that is the combined framework regions of the constituent light and heavy chains, serves to position and align the CDRs in three dimensional spaces.

The CDRs are primarily responsible for binding to an epitope of an antigen. The CDRs of each chain are typically referred to as CDR1, CDR2, and CDR3, numbered sequentially starting from the N-terminus, and are also typically identified by the chain in which the particular CDR is located. Thus, a V_{H} CDR3 is located in the variable domain of the heavy chain of the antibody in which it is found, whereas a V_{L} CDR1 is the CDR1 from the variable domain of the light chain of the antibody in which it is found.
References to "V_{H}" refer to the variable region of an immunoglobulin heavy chain of an antibody, including the heavy chain of an Fv, scFv, or Fab. References to "V_{L}" refer to the variable region of an immunoglobulin light chain, including the light chain of an Fv, scFv, dsFv or Fab.

The phrase "single chain Fv" or "scFv" refers to an antibody in which the variable domains of the heavy chain and of the light chain of a traditional two chain antibody have been joined to form one chain. Typically, a linker peptide is inserted between the two chains to allow for proper folding and creation of an active binding site.

A "chimeric antibody" is an immunoglobulin molecule in which (a) the constant region, or a portion thereof, is altered, replaced or exchanged so that the antigen binding site (variable region) is linked to a constant region of a different or altered class, effector function and/or species, or an entirely different molecule which confers new properties to the chimeric antibody, *e.g*., an enzyme, toxin, hormone, growth factor, drug, *etc*.; or (b) the variable region, or a portion thereof, is altered, replaced or exchanged with a variable region having a different or altered antigen specificity.

A "humanized antibody" is an immunoglobulin molecule that contains minimal sequence derived from non-human immunoglobulin. Humanized antibodies include human immunoglobulins (recipient antibody) in which residues from a complementary determining region (CDR) of the recipient are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues. Humanized antibodies may also comprise residues which are found neither in the recipient antibody nor in the imported CDR or framework sequences. In general, a humanized antibody will comprise substantially all of at least one, and typically two, variable domains, in which all or substantially all of the CDR regions correspond to those of a non-human immunoglobulin and all or substantially all of the framework (FR) regions are those of a human immunoglobulin consensus sequence. The humanized antibody optimally also will comprise at least a portion of an immunoglobulin constant region (Fc), typically that of a human immunoglobulin (Jones et al., Nature 321:522-525 (1986); Riechmann et al., Nature 332:323-327 (1988); and Presta, Curr. Op. Strut. Biol. 2:593-596 (1992)). Humanization can be essentially performed following the method of Winter and co-workers (Jones et al., Nature 321:522-525 (1986); Riechmann et al., Nature 332:323-327 (1988); Verhoeyen et al., Science 239:1534-1536 (1988)), by substituting rodent CDRs or CDR sequences for the corresponding sequences of a human antibody. Accordingly, such humanized antibodies are chimeric antibodies (U.S. Patent No. 4,816,567), wherein substantially less than an intact human variable domain has been substituted by the corresponding sequence from a non-human species.

The terms "epitope" and "antigenic determinant" refer to a site on an antigen to which an antibody binds. Epitopes can be formed both from contiguous amino acids or noncontiguous amino acids juxtaposed by tertiary folding of a protein. Epitopes formed from contiguous amino acids are typically retained on exposure to denaturing solvents whereas epitopes formed by tertiary folding are typically lost on treatment with denaturing solvents. An epitope typically includes at least 3, and more usually, at least 5 or 8-10 amino acids in a unique spatial conformation. Methods of determining spatial conformation of epitopes include, for example, x-ray crystallography and 2-dimensional nuclear magnetic resonance. See, *e.g*., Epitope Mapping Protocols in Methods in Molecular Biology, Vol. 66, Glenn E. Morris, Ed (1996).

The terms "polypeptide," "peptide" and "protein" are used interchangeably herein to refer to a polymer of amino acid residues. The terms apply to amino acid polymers in which one or more amino acid residue is an artificial chemical mimetic of a corresponding naturally occurring amino acid, as well as to naturally occurring amino acid polymers, those containing modified residues, and non-naturally occurring amino acid polymer.

The term "amino acid" refers to naturally occurring and synthetic amino acids, as well as amino acid analogs and amino acid mimetics that function similarly to the naturally occurring amino acids. Naturally occurring amino acids are those encoded by the genetic code, as well as those amino acids that are later modified, *e.g*., hydroxyproline, γ-carboxyglutamate, and O-phosphoserine. Amino acid analogs refers to compounds that have the same basic chemical structure as a naturally occurring amino acid, *e.g*., an α carbon that is bound to a hydrogen, a carboxyl group, an amino group, and an R group, *e.g*., homoserine, norleucine, methionine sulfoxide, methionine methyl sulfonium. Such analogs may have modified R groups (*e.g*., norleucine) or modified peptide backbones, but retain the same basic chemical structure as a naturally occurring amino acid. Amino acid mimetics refers to chemical compounds that have a structure that is different from the general chemical structure of an amino acid, but that functions similarly to a naturally occurring amino acid.

Amino acids may be referred to herein by their commonly known three letter symbols or by the one-letter symbols recommended by the IUPAC-IUB Biochemical Nomenclature Commission. Nucleotides, likewise, may be referred to by their commonly accepted single-letter codes.

The term "recombinant" when used with reference, *e.g*., to a cell, or nucleic acid, protein, or vector, indicates that the cell, nucleic acid, protein or vector, has been modified by the introduction of a heterologous nucleic acid or protein or the alteration of a native nucleic acid or protein, or that the cell is derived from a cell so modified. Thus, *e.g*., recombinant cells express genes that are not found within the native (non-recombinant) form of the cell or express native genes that are otherwise abnormally expressed, under expressed or not expressed at all. By the term "recombinant nucleic acid" herein is meant nucleic acid, originally formed *in vitro,* in general, by the manipulation of nucleic acid, *e.g*., using polymerases and endonucleases, in a form not normally found in nature. In this manner, operable linkage of different sequences is achieved. Thus an isolated nucleic acid, in a linear form, or an expression vector formed *in vitro* by ligating DNA molecules that are not normally joined, are both considered recombinant for the purposes of this invention. It is understood that once a recombinant nucleic acid is made and reintroduced into a host cell or organism, it will replicate non-recombinantly, *i*.*e*., using the *in vivo* cellular machinery of the host cell rather than *in vitro* manipulations; however, such nucleic acids, once produced recombinantly, although subsequently replicated non-recombinantly, are still considered recombinant for the purposes of the invention. Similarly, a "recombinant protein" is a protein made using recombinant techniques, *i*.*e*., through the expression of a recombinant nucleic acid as depicted above.

Unless otherwise defined, all technical and scientific terms used herein have the same meaning as commonly understood by one of ordinary skill in the art to which this invention belongs. In case of conflict, the present specification, including definitions, will control.

### III. Producing and identifying compounds to treat ANLN-mediated disease

In view of the evidence provided herein, that ANLN interacts with RhoA and ANLN expression is associated with poor prognosis in cancer patients, one aspect of the invention involves identifying test compounds that reduce or prevent the binding between ANLN and RhoA. Moreover, in view of the evidence provided herein, that expression of ANLN is associated with increased cell motility, methods of identifying test compounds that inhibit ANLN-mediated motility are described.

Methods for determining ANLN/RhoA binding include any methods for determining interactions of two proteins. Such assays include, but are not limited to, traditional approaches, such as, cross-linking, co-immunoprecipitation, and co-purification through gradients or chromatographic columns. In addition, protein-protein interactions can be monitored using a yeast-based genetic system described by Fields and co-workers (Fields and Song, Nature 340:245-246 (1989); Chien et al., Proc. Natl. Acad. Sci. USA 88, 9578-9582 (1991)) and as disclosed by Chevray and Nathans (Proc. Natl. Acad. Sci. USA 89:5789-5793 (1992)). Many transcriptional activators, such as yeast GALA, consist of two physically discrete modular domains, one acting as the DNA-binding domain, while the other one functions as the transcription activation domain. The yeast expression system described in the foregoing publications (generally referred to as the "two-hybrid system") takes advantage of this property, and employs two hybrid proteins, one in which the target protein is fused to the DNA-binding domain of GAL4, and another, in which candidate activating proteins are fused to the activation domain. The expression of a GAL1-lacZ reporter gene under control of a GALA-activated promoter depends on reconstitution of GAL4 activity via protein-protein interaction. Colonies containing interacting polypeptides are detected with a chromogenic substrate for β-galactosidase. A complete kit (MATCHMAKER™) for identifying protein-protein interactions between two specific proteins using the two-hybrid technique is commercially available from Clontech. This system can also be extended to map protein domains involved in specific protein interactions as well as to pinpoint amino acid residues that are crucial for these interactions.

While the present application refers to "ANLN" and "RhoA," it is understood that where the interaction of the two is analyzed or manipulated, it is possible to use the binding portions of one or both of the proteins in place of the full-length copies of the proteins. Fragments of ANLN that bind to RhoA may be readily identified using standard deletion analysis and/or mutagenesis of ANLN to identify fragments that bind to RhoA. Similar analysis may be used to identify ANLN-binding fragments of RhoA.

Methods of identifying test compounds that inhibit ANLN-mediated cell motility may be performed by contacting cells expressing ANLN with a compound and then observing cell motility. Any mammalian cells derived from normal or cancer tissues may be used, for example, NIH3T3, COS-7, HEK293, SAEC, BEAS-2B cells. Cell motility can be determined using standard assays such as Matrigel invasion assays.

As disclosed herein, any test compounds, including, *e.g*., proteins (including antibodies), muteins, polynucleotides, nucleic acid aptamers, and peptide and nonpeptide small organic molecules, may serve as test compounds of the present invention. Test compounds may be isolated from natural sources, prepared synthetically or recombinantly, or any combination of the same.

For example, peptides may be produced synthetically using solid phase techniques as described in "Solid Phase Peptide Synthesis" by G. Barany and R. B. Merrifield in Peptides, Vol. 2, edited by E. Gross and J. Meienhoffer, Academic Press, New York, N.Y., pp. 100-118 (1980). Similarly, nucleic acids can also be synthesized using the solid phase techniques, as described in Beaucage, S.L., & Iyer, R.P. (1992) Tetrahedron, 48, 2223-2311; and Matthes et al., EMBO J., 3:801-805 (1984).

Where inhibitory peptides are identified, modifications of peptides of the present invention with various amino acid mimetics or unnatural amino acids are particularly useful in increasing the stability of the peptide *in vivo.* Stability can be assayed in a number of ways. For instance, peptidases and various biological media, such as human plasma and serum, have been used to test stability. *See, e*.*g*., Verhoef et al., Eur. J. Drug Metab Pharmacokin. 11:291-302 (1986). Other useful peptide modifications known in the art include glycosylation and acetylation.

Both recombinant and chemical synthesis techniques may be used to produce test compounds of the present invention. For example, a nucleic acid test compound may be produced by insertion into an appropriate vector, which may be expanded when transfected into a competent cell. Alternatively, nucleic acids may be amplified using PCR techniques or expression in suitable hosts (cf. Sambrook et al., Molecular Cloning: A Laboratory Manual, 1989, Cold Spring Harbor Laboratory, New York, USA).

Peptides and proteins may also be expressed using recombinant techniques well known in the art, *e.g*., by transforming suitable host cells with recombinant DNA constructs as described in Morrison, J. Bact., 132:349-351 (1977); and Clark-Curtiss & Curtiss, Methods in Enzymology, 101:347-362 (1983).

### Anti- ANLN and anti-RhoA antibodies

In some aspects of the present invention, test compounds are anti-ANLN or anti-RhoA antibodies. In some embodiments, the antibodies are chimeric, including but not limited to, humanized antibodies. In some cases, antibody embodiments of the present invention will bind either ANLN or RhoA at the interface where one of these proteins associates with the other. In some embodiments, these antibodies bind ANLN or RhoA with a Kₐ of at least about 10⁵ mor⁻¹, 10⁶ mol⁻¹ or greater, 10⁷ mol⁻¹ or greater, 10⁸ mol⁻¹ or greater, or 10⁹ mol⁻¹ or greater under physiological conditions. Such antibodies can be purchased from a commercial source, for example, Chemicon, Inc. (Temecula, CA), or can be raised using as an immunogen, such as a substantially purified ANLN or RhoA protein, *e.g*., a human protein, or an antigenic fragment thereof. Methods of preparing both monoclonal and polyclonal antibodies from provided immunogens are well-known in the art. For purification techniques and methods for identifying antibodies to specific immunogens, *see e.g.,* PCT/LTS02/07144 (WO/03/077838) . Methods for purifying antibodies using, for example, antibody affinity matrices to form an affinity column are also well known in the art and available commercially (AntibodyShop, Copenhagen, Denmark). Identification of antibodies capable of disrupting ANLN/RhoA association is performed using the same test assays detailed below for test compounds in general.

### Converting enzymes

Converting enzymes may act as test compounds of the present invention. In the context of the present invention, converting enzymes are molecular catalysts that perform covalent post-translational modifications to either ANLN, RhoA, or both. Converting enzymes of the present invention will covalently modify one or more amino acid residues of ANLN and/or RhoA in a manner that causes either an allosteric alteration in the structure of the modified protein, or alters the ANLN/RhoA molecular binding site chemistry or structure of the modified protein in a manner that interferes with binding between ANLN and RhoA. Herein, interference with binding between the two molecules refers to a decrease in the Kₐ of binding by at least 25%, 30%, 40%, 50%, 60%, 70% or more relative to the Ka of binding between the proteins measured at 30°C and an ionic strength of 0.1 in the absence of detergents. Exemplary converting enzymes of the invention include kinases, phosphatases, amidases, acetylases, glycosidase and the like.

### Constructing test compound libraries

Although the construction of test compound libraries is well known in the art, the present section provides additional guidance in identifying test compounds and construction libraries of such compounds for screening of effective inhibitors of ANLN/RhoA interaction and/or ANLN-mediated cell motility.

### Molecular modeling

Construction of test compound libraries is facilitated by knowledge of the molecular structure of compounds known to have the properties sought, and/or the molecular structure of the target molecules to be inhibited, *i*.*e*., ANLN and RhoA. One approach to preliminary screening of test compounds suitable for further evaluation is computer modeling of the interaction between the test compound and its target. In the present invention, modeling the interaction between ANLN and/or RhoA provides insight into both the details of the interaction itself, and suggests possible strategies for disrupting the interaction, including potential molecular inhibitors of the interaction.

Computer modeling technology allows visualization of the three-dimensional atomic structure of a selected molecule and the rational design of new compounds that will interact with the molecule. The three-dimensional construct typically depends on data from x-ray crystallographic analysis or NMR imaging of the selected molecule. The molecular dynamics require force field data. The computer graphics systems enable prediction of how a new compound will link to the target molecule and allow experimental manipulation of the structures of the compound and target molecule to perfect binding specificity. Prediction of what the molecule-compound interaction will be when small changes are made in one or both requires molecular mechanics software and computationally intensive computers, usually coupled with user-friendly, menu-driven interfaces between the molecular design program and the user.

An example of the molecular modeling system described generally above consists of the CHARMm and QUANTA programs, Polygen Corporation, Waltham, Mass. CHARMm performs the energy minimization and molecular dynamics functions. QUANTA performs the construction, graphic modeling and analysis of molecular structure. QUANTA allows interactive construction, modification, visualization, and analysis of the behavior of molecules with each other.

A number of articles review computer modeling of drugs interactive with specific proteins, such as Rotivinen, et al., Acta Pharmaceutica Fennica 97, 159-166 (1988); Ripka, New Scientist 54-57 (Jun. 16, 1988); McKinlay and Rossmann, Annu. Rev. Pharmacol. Toxiciol. 29, 111-122 (1989); Perry and Davies, Prog Clin Biol Res. 291:189-93(1989); Lewis and Dean, Proc. R. Soc. Lond. 236, 125-140 and 141-162 (1989); and, with respect to a model receptor for nucleic acid components, Askew, et al., J. Am. Chem. Soc. 111, 1082-1090 (1989).

Other computer programs that screen and graphically depict chemicals are available from companies such as BioDesign, Inc., Pasadena, Calif., Allelix, Inc, Mississauga, Ontario, Canada, and Hypercube, Inc., Cambridge, Ontario. *See, e*.*g*., DesJarlais et al. (1988) J. Med. Chem. 31:722; Meng et al. (1992) J. Computer Chem. 13:505; Meng et al. (1993) Proteins 17:266; Shoichet et al. (1993) Science 259:1445.

Once a putative inhibitor of the ANLN/RhoA interaction has been identified, combinatorial chemistry techniques can be employed to construct any number of variants based on the chemical structure of the identified putative inhibitor, as detailed below. The resulting library of putative inhibitors, or "test compounds" may be screened using the methods described herein to identify test compounds of the library that disrupt the ANLN/RhoA association.

### Combinatorial chemical syntheses

Combinatorial libraries of test compounds may be produced as part of a rational drug design program involving knowledge of core structures existing in known inhibitors of the ANLN/RhoA interaction. This approach allows the library to be maintained at a reasonable size, facilitating high throughput screening. Alternatively simple, particularly short polymeric molecular libraries may be constructed by simply synthesizing all permutations of the molecular family making up the library. An example of this latter approach would be a library of all peptides six amino acids in length. Such a peptide library could include every 6 amino acid sequence permutation. This type of library is termed a linear combinatorial chemical library.

Preparation of combinatorial chemical libraries is well known to those of skill in the art, and may be generated by either chemical or biological synthesis. Combinatorial chemical libraries include, but are not limited to, peptide libraries (*see, e.g.,* U.S. Patent 5,010,175, Furka, Int. J. Pept. Prot. Res. 37:487-493 (1991) and Houghten et al., Nature 354:84-86 (1991)). Other chemistries for generating chemical diversity libraries can also be used. Such chemistries include, but are not limited to: peptides (*e.g*., PCT Publication No. WO 91/19735), encoded peptides (*e.g*., PCT Publication WO 93/20242), random bio-oligomers (*e.g*., PCT Publication No. WO 92/00091), benzodiazepines (*e.g*., U.S. Pat. No. 5,288,514), diversomers such as hydantoins, benzodiazepines and dipeptides (DeWitt et al., Proc. Natl. Acad Sci. USA 90:6909-6913 (1993)), vinylogous polypeptides (Hagihara et al., J. Amer. Chem. Soc. 114:6568 (1992)), nonpeptidal peptidomimetics with glucose scaffolding (Hirschmann et al., J. Amer. Chem. Soc. 114:9217-9218 (1992)), analogous organic syntheses of small compound libraries (Chen et al., J. Amer. Chem. Soc. 116:2661 (1994)), oligocarbamates (Cho et al., Science 261:1303 (1993)), and/or peptidyl phosphonates (Campbell et al., J. Org. Chem. 59:658 (1994)), nucleic acid libraries *(see* Ausubel, and Sambrook, all *supra*), peptide nucleic acid libraries (*see, e.g.,* U.S. Patent 5,539,083), antibody libraries (*see, e.g.,* Vaughan et al., Nature Biotechnology, 14(3):309-314 (1996) and PCT/US96/10287), carbohydrate libraries (*see, e.g*., Liang et al., Science, 274:1520-1522 (1996) and U.S. Patent 5,593,853), small organic molecule libraries (*see, e.g.,* benzodiazepines, Baum C&EN, Jan 18, page 33 (1993); isoprenoids, U.S. Patent 5,569,588; thiazolidinones and metathiazanones, U.S. Patent 5,549,974; pyrrolidines, U.S. Patents 5,525,735 and 5,519,134; morpholino compounds, U.S. Patent 5,506,337; benzodiazepines, 5,288,514, and the like).

### Phage display

Another approach uses recombinant bacteriophage to produce libraries. Using the "phage method" (Scott and Smith, Science 249:386-390, 1990; Cwirla, et al, Proc. Natl. Acad. Sci., 87:6378-6382, 1.990; Devlin et al., Science, 249:404-406, 1990), very large libraries can be constructed (*e*.*g*., 10⁶ -10⁸ chemical entities). A second approach uses primarily chemical methods, of which the Geysen method (Geysen et al., Molecular Immunology 23:709-715, 1986; Geysen et al. J. Immunologic Method 102:259-274, 1987; and the method of Fodor et al. (Science 251:767-773, 1991) are examples. Furka et al. (14th International Congress of Biochemistry, Volume #5, Abstract FR:013, 1988; Furka, Int. J. Peptide Protein Res. 37:487-493, 1991), (U.S. Pat. No. 4,631,211) and. (U.S. Pat. No. 5,010,175) describe methods to produce a mixture of peptides that can be tested as agonists or antagonists.

Devices for the preparation of combinatorial libraries are commercially available (*see, e*.*g*., 357 MPS, 390 MPS, Advanced ChemTech, Louisville KY, Symphony, Rainin, Woburn, MA, 433A Applied Biosystems, Foster City, CA, 9050 Plus, Millipore, Bedford, MA). In addition, numerous combinatorial libraries are themselves commercially available (*see, e.g.,* ComGenex, Princeton, N.J., Tripos, Inc., St. Louis, MO, 3D Pharmaceuticals, Exton, PA, Martek Biosciences, Columbia, MD, *etc*.).

### Screening test compound libraries

Screening methods described herein provide efficient and rapid identification of test compounds that have a high probability of interfering with the ANLN/RhoA association or with ANLN-mediated cell motility. Generally, any method that determines the ability of a test compound to interfere with the ANLN/RhoA association or ANLN-mediated cell motility is suitable. For example, competitive and non-competitive inhibition assays in an ELISA format may be utilized. Control experiments should be performed to determine maximal binding capacity of system (*e.g*., contacting bound ANLN with RhoA and determining the amount of RhoA that binds to ANLN in the examples below).

### Competitive assay format

Competitive assays may be used for screening test compounds. By way of example, a competitive ELISA format may include ANLN (or RhoA) bound to a solid support. The bound ANLN (or RhoA) would be incubated with RhoA (or ANLN) and a test compound. After sufficient time to allow the test compound and/or RhoA (or ANLN) to bind ANLN (or RhoA), the substrate would be washed to remove unbound material. The amount of RhoA (or ANLN) bound to ANLN (or RhoA) is then determined. This may be accomplished in any of a variety of ways known in the art, for example, by using an RhoA (or ANLN) species tagged with a detectable label, or by contacting the washed substrate with a labeled anti-RhoA (or ANLN) antibody. The amount of RhoA (or ANLN) bound to ANLN (or RhoA) will be inversely proportional to the ability of the test compound to interfere with the RhoA/ANLN association. Protein, including but not limited to, antibody, labeling is described in Harlow & Lane, Antibodies, A Laboratory Manual (1988).

In a variation, ANLN (or RhoA) is labeled with an affinity tag. Labeled ANLN (or RhoA) is then incubated with a test compound and RhoA (or ANLN), then immunoprecipitated. The immunoprecipitate is then subjected to Western blotting using an anti-RhoA (or ANLN) antibody. As with the previous competitive assay format, the amount of RhoA (or ANLN) found associated with ANLN (or RhoA) is inversely proportional to the ability of the test compound to interfere with the ANLN/RhoA association.

### Non-competitive assay format

Non-competitive binding assays may also find utility as an initial screen for test compound libraries constructed in a format that is not readily amenable to screening using competitive assays, such as those described herein. An example of such a library is a phage display library (*See, e*.*g*., Barrett, et al. (1992) Anal. Biochem 204,357-364).

Phage libraries find utility in being able to produce quickly working quantities of large numbers of different recombinant peptides. Phage libraries do not lend themselves to competitive assays of the invention, but can be efficiently screened in a non-competitive format to determine which recombinant peptide test compounds bind ANLN or RhoA. Test compounds identified as binding can then be produced and screened using a competitive assay format. Production and screening of phage and cell display libraries is well-known in the art and discussed in, for example, Ladner et al., WO 88/06630; Fuchs et al. (1991) Biotechnology 9:1369-1372; Goward et al. (1993) TIBS 18:136-140; Charbit et al. (1986) EMBO J 5, 3029-3037. Cull et al. (1992) PNAS USA 89:1865-1869; Cwirla, et al. (1990) Proc. Natl. Acad. Sci. U.S.A. 87, 6378-6382.

An exemplary non-competitive assay would follow an analogous procedure to the one described for the competitive assay, without the addition of one of the components (ANLN or RhoA). However, as non-competitive formats determine test compound binding to ANLN or RhoA, the ability of test compound to both ANLN and RhoA needs to be determined for each candidate. Thus, by way of example, binding of the test compound to immobilized ANLN may be determined by washing away unbound test compound; eluting bound test compound from the support, followed by analysis of the eluate; *e*.*g*., by mass spectroscopy, protein determination (Bradford or Lowry assay, or Abs. at 280nm determination.). Alternatively, the elution step may be eliminated and binding of test compound determined by monitoring changes in the spectroscopic properties of the organic layer at the support surface. Methods for monitoring spectroscopic properties of surfaces include, but are not limited to, absorbance, reflectance, transmittance, birefringence, refractive index, diffraction, surface plasmon resonance, ellipsometry, resonant mirror techniques; grating coupled waveguide techniques and multipolar resonance spectroscopy, all of which are known to those of skill in the art. A labeled test compound may also be used in the assay to eliminate need for an elution step. In this instance, the amount of label associated with the support after washing away unbound material is directly proportional to test compound binding.
A number of well-known robotic systems have been developed for solution phase chemistries. These systems include automated workstations like the automated synthesis apparatus developed by Takeda Chemical Industries, LTD. (Osaka, Japan) and many robotic systems utilizing robotic arms (Zymate II, Zymark Corporation, Hopkinton, Mass.; Orca, Hewlett Packard, Palo Alto, Calif.), which mimic the manual synthetic operations performed by a chemist. Any of the above devices are suitable for use with the present invention. The nature and implementation of modifications to these devices (if any) so that they can operate as discussed herein will be apparent to persons skilled in the relevant art. In addition, numerous combinatorial libraries are themselves commercially available (*see, e*.*g.,* ComGenex, Princeton, N.J., Asinex, Moscow, Ru, Tripos, Inc., St. Louis, MO, ChemStar, Ltd, Moscow, RU, 3D Pharmaceuticals, Exton, PA, Martek Biosciences, Columbia, MD, *etc*.).

### Screening converting enzymes

Test compounds that are converting enzymes may be assayed in a noncompetitive format, using co-factors and auxiliary substrates specific for the converting enzyme being assayed. Such co-factors and auxiliary substrates are known to one of skill in the art, given the type of converting enzyme to be investigated.

One exemplary screening procedure for converting enzymes involves first contacting ANLN and/or RhoA with the converting enzyme in the presence of co-factors and auxiliary substrates necessary to perform covalent modification of the protein characteristic of the converting enzyme, preferably under physiologic conditions. The modified protein(s) is then tested for its ability to bind to its binding partner (*i*.*e*., binding of ANLN to RhoA). Binding of the modified protein to its binding partner is then compared to binding of unmodified control pairs to determine if the requisite change in Kₐ noted above has been achieved.

To facilitate the detection of proteins in performing the assay, one or more proteins may be labeled with a detectable label as described above, using techniques well known to those of skill in the art.

### Methods for Screens

The screening embodiments described above are suitable for high through-put determination of test compounds suitable for further investigation. The screening method described herein may involve the detection of one or more of the following:
(a) the concentration of activated RhoA;
(b) the interaction between RhoA and an RHO effector (*e*.*g*., ROCK (Maekawa, M. et.al. Science vol 285. 895-898(1999)), Rhophilin-2 (Jeremy W. Peck et. al. Journal of Biological Chemistry 277, 43 924-32(2002)) or RhoA binding region thereof;
(c) the activation of any signal complex including downstream gene expression (*e*.*g*., ROCK (Stephan A.K. Harvey et.al. Investigative Ophthalmology & Visual Science 45, 2168-76(2004)), c-JUN(Maria Julia Marinissen et.al. Molecular Cell14, 29-41(2004)) or downstream gene product activity (*e*.*g*., ERK(Laboureau J, et.al. Experimental Dermatology 13, 70-7(2004)), GATA-4(Yanazume T, et.al. The Journal of Biological Chemistry 277, 8618-25(2002)) mediated by activated RhoA;
(d) the promotion of DNA synthesis and cell cycle entry;
(e) cell migration or any other oncogenic phenotype cell adhesion, cell invasion (Shibata, T et.al. American Journal of Pathology 164, 2269-78(2004), Selma Cetin et.al. The Journal of Biological Chemistry 279, 24592-600(2004));
(f) actin stress fiber formation and F-actin production; and
(g) the interaction with any molecules important for cell adhesion, migration and invasion (Spred (Miyoshi, K. et.al. Oncogene 23, 5567-5576(2004)), Smurf1 (Hong-Rui Wang et.al. Science 302, 1775-9(2003))).

Alternatively, the test compound under investigation may be added to proliferating cells and proliferation of the treated cells monitored relative to proliferation of a control population not supplemented with the test compound. Cell lines suitable for screening test compounds will be obvious to one of skill in the art provided with the teachings presented herein.

For *in vivo* testing, the test compound may be administered to an accepted animal model.

### IV. Formulating medicaments from identified test compounds

Accordingly, the present invention describes medicaments and methods useful in preventing or treating cancer, particularly a lung cancer such as non-small cell lung cancer, as well as other cancers characterized by cells displaying elevated the expression levels and the activity of ANLN and/or RhoA and/or nuclear localization of ANLN. These medicaments and methods comprise at least one test compound of the present invention identified as disruptive to the ANLN/RhoA interaction in an amount effective to achieve attenuation or arrest of pathologic cell proliferation. More specifically, a therapeutically effective amount means an amount effective to prevent the development of or to alleviate existing symptoms of the subject being treated.

Individuals to be treated with methods of the present invention may be any individual afflicted with cancer, including, *e*.*g*., non-small cell lung cancer characterized by elevated expression of marker protein ANLN or exhibiting nuclear localization of ANLN. Such an individual can be, for example, a vertebrate such as a mammal, including a human, dog, cat, horse, cow, or goat; or any other animal, particularly a commercially important animal or a domesticated animal. For purposes of the present invention, elevated expression of marker proteins refers to a mean cellular marker protein concentration for one or both marker proteins that is at least 10%, preferably 15%, 20%, 25%, 30%, 35%, 40%, 45%, 50%, 55% or more above normal mean cellular concentration of the marker protein(s).

### Determining therapeutic dose range

Determination of an effective dose range for the medicaments as described herein is well within the capability of those skilled in the art, especially in light of the detailed disclosure provided herein. The therapeutically effective dose for a test compound can be estimated initially from cell culture assays and/or animal models. For example, a dose can be formulated in animal models to achieve a circulating concentration range that includes the IC₅₀ (the dose where 50% of the cells show the desired effects) as determined in cell culture. Toxicity and therapeutic efficacy of test compounds also can be determined by standard pharmaceutical procedures in cell cultures or experimental animals, *e*.*g*., for determining the LD₅₀ (the dose lethal to 50% of the population) and the ED₅₀ (the dose therapeutically effective in 50% of the population). The dose ratio between toxic and therapeutic effects is the therapeutic index (*i*.*e*., the ratio between LD₅₀ and ED₅₀). Compounds which exhibit high therapeutic indices may be used. The data obtained from these cell culture assays and animal studies may be used in formulating a dosage range for use in humans. The dosage of such compounds may lie within a range of circulating concentrations that include the ED₅₀ with little or no toxicity. The dosage may vary within this range depending upon the dosage form employed and the route of administration utilized. The exact formulation, route of administration and dosage can be chosen by the individual physician in view of the patient's condition. *See, e*.*g*., Fingl *et al.,* (1975), in "The Pharmacological Basis of Therapeutics", Ch. 1 p1. Dosage amount and interval may be adjusted individually to provide plasma levels of the active test compound sufficient to maintain the desired effects.

### Pharmaceutically acceptable excipients

Medicaments administered to a mammal (*e*.*g*., a human) may contain a pharmaceutically-acceptable excipient, or carrier. Suitable excipients and their formulations are described in Remington's Pharmaceutical Sciences, 16th ed., (1980), Mack Publishing Co., edited by Oslo *et al*. For aqueous preparations an appropriate amount of a pharmaceutically-acceptable salt is typically used in the formulation to render the formulation isotonic. Examples of the pharmaceutically-acceptable isotonic excipients include liquids such as saline, Ringer's solution, Hanks's solution and dextrose solution. Isotonic excipients are particularly important for injectable formulations.

For transmucosal administration, penetrants appropriate to the barrier to be permeated are used in the formulation. Such penetrants are generally known in the art.

Excipients may be used to maintain the correct pH of the formulation. For optimal shelf life, the pH of solutions containing test compounds preferably ranges from about 5 to about 8, and more preferably from about 7 to about 7.5. The formulation may also comprise a lyophilized powder or other suitable optional excipients including sustained release preparations such as semi-permeable matrices of solid hydrophobic polymers, which matrices are in the form of shaped articles, *e*.*g*., films, liposomes or microparticles. It will be apparent to those persons skilled in the art that certain excipients may be more preferable depending upon, for instance, the route of administration, the concentration of test compound being administered, or whether the treatment uses a medicament that includes a protein, a nucleic acid encoding the test compound, or a cell capable of secreting a test compound as the active ingredient.

The pharmaceutical compositions may be manufactured in a manner that is itself known, *e*.*g*., by means of conventional mixing, dissolving, granulating, dragee-making, levigating, emulsifying, encapsulating, entrapping or lyophilizing processes. Proper formulation is dependent upon the route of administration chosen.

For oral administration, carriers enable the compounds to be formulated as tablets, pills, dragees, capsules, liquids, gels, syrups, slurries, suspensions and the like, for oral ingestion by a patient to be treated. Pharmaceutical preparations for oral use can be obtained by formulating a test compound with a solid dispersable excipient, optionally grinding a resulting mixture and processing the mixture of granules after adding suitable auxiliaries, if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl-cellulose, sodium carboxymethylcellulose, and/or polyvinylpyrrolidone (PVP). If desired, disintegrating agents may be added, such as the cross-linked polyvinyl pyrrolidone, agar, or alginic acid or a salt thereof such as sodium alginate.

Many of the compound described herein may be optionally provided as salts with pharmaceutically compatible counterions. Pharmaceutically compatible salts may be formed with many acids, including but not limited to hydrochloric, sulfuric, acetic, lactic, tartaric, malic, succinic, etc, depending upon the application. Salts tend to be more soluble in aqueous or other protonic solvents that are the corresponding free base forms.

In addition to acceptable excipients, formulations described herein may include therapeutic agents other than identified test compounds. For example formulations may include anti-inflammatory agents, pain killers, chemotherapeutics, mucolytics (*e*.*g*. n-acetylcysteine) and the like. In addition to including other therapeutic agents in the medicament itself, the medicaments may also be administered sequentially or concurrently with the one or more other pharmacologic agents. The amounts of medicament and pharmacologic agent depend, for example, on what type of pharmacologic agent(s) is are used, the disease being treated, and the scheduling and routes of administration.

Following administration of a medicament, the mammal's physiological condition can be monitored in various ways well known to the skilled practitioner.

### Gene Therapy

Protein and peptide test compounds identified as disruptors of the ANLN/RhoA association may be therapeutically delivered using gene therapy to patients suffering from cancer, *e*.*g*., non-small cell lung cancer. Exemplary test compounds amenable to gene therapy techniques include, but are not limited to, converting enzymes as well as peptides that directly alter the ANLN/RhoA association by steric or allosteric interference. In some aspects, gene therapy embodiments include a nucleic acid sequence encoding a suitable identified test compound of the invention. In some embodiments, the nucleic acid sequence includes those regulatory elements necessary for expression of the test compound in a target cell. The nucleic acid may be equipped to stably insert into the genome of the target cell (*see e.g.,* Thomas, K. R. and Capecchi, M. R. (1987) Cell 51:503 for a description of homologous recombination cassettes vectors).

Delivery of the nucleic acids into a patient may be either direct, in which case the patient is directly exposed to the nucleic acid or nucleic acid-carrying vectors, or indirect, in which case, cells are first transformed with the nucleic acids *in vitro,* then transplanted into the patient. These two approaches are known, respectively, as *in vivo* or *ex vivo* gene therapy.

For general reviews of the methods of gene therapy, *see* Goldspiel et al., 1993, Clinical Pharmacy 12:488-505; Wu and Wu, 1991, Biotherapy 3:87-95; Tolstoshev, 1993, Ann. Rev. Pharmacol. Toxicol. 33:573-596; Mulligan, 1993, Science 260:926-932; and Morgan and Anderson, 1993, Ann. Rev. Biochem. 62:191-217; 1993, TIBTECH 11(5):155-215). Methods commonly known in the art of recombinant DNA technology which can be used are described in Ausubel et al. (eds.), 1993, Current Protocols in Molecular Biology, John Wiley & Sons, NY; and Kriegler, 1990, Gene Transfer and Expression, A Laboratory Manual, Stockton Press, NY.

### V. Screening, Prognosis and Treatment Kits

The present invention also describes an article of manufacture or kit containing materials for screening for a compound useful in treating or preventing cancer, particularly a lung cancer such as non-small cell lung cancer (NSCLC). Such an article of manufacture may comprise one or more labeled containers of materials described herein along with instructions for use. Suitable containers include, for example, bottles, vials, and test tubes. The containers may be formed from a variety of materials such as glass or plastic.

In one embodiment, the screening kit comprises: (a) a first polypeptide comprising an RhoA-binding domain of an ANLN polypeptide; (b) a second polypeptide comprising an ANLN-binding domain of an RhoA polypeptide, and (c) means (*e*.*g*., a reagent) to detect the interaction between the first and second polypeptides.

In some embodiments, the first polypeptide, *i*.*e*., the polypeptide comprising the RhoA-binding domain, comprises an ANLN polypeptide. Similarly, in other embodiments, the second polypeptide, *i*.*e*., the polypeptide comprising the ANLN-binding domain, comprises an RhoA polypeptide.

In some embodiments, the polypeptide comprising an RhoA-binding domain is expressed in a living cell.

In some embodiments, the means (*e*.*g*., the reagent) to detect the interaction between the two polypeptides can detect:
(1) the concentration of activated RhoA;
(2) the interaction between RhoA and an RHO effector or an RhoA-binding region thereof;
(3) the activation of any signal complex, including downstream genes mediated by activated RhoA;
(4) the promotion of DNA synthesis and cell cycle entry;
(5) cell migration or any other oncogenic phenotype;
(6) actin stress fiber formation and F-actin production; and
(7) the interaction with any molecules important for cell adhesion, migration and invasion.

In another embodiment, the screening kit may comprise: (a) a cell expressing an ANLN polypeptide or a functional equivalent thereof; and (b) means (*e*.*g*., a reagent) to detect the motility of the cell.

The present invention also describes kits for predicting the prognosis of a cancer subject, for example a subject afflicted with a lung cancer such as non-small cell lung cancer (NSCLC). In some embodiments, such prognosis kits comprise: (a) an antibody recognizing an ANLN protein and (b) an agent for detection in the nucleus. In some embodiments, the agent for detection of the nucleus is hematoxylin-eosin staining dye. Instructions for use would indicate that detection of ANLN localized in the nucleus of a specimen collected from a subject whose NSCLC prognosis is to be predicted is indicative of poor prognosis.

The present invention further describes articles of manufacture and kits containing materials useful for treating the pathological conditions described herein are provided. Such an article of manufacture may comprise a container of a medicament as described herein with a label. As noted above, suitable containers include, for example, bottles, vials, and test tubes. The containers may be formed from a variety of materials such as glass or plastic. In the context of the present invention, the container holds a composition having an active agent which is effective for treating a cell proliferative disease, for example, non-small cell lung cancer. The active agent in the composition may be an identified test compound (*e*.*g*., antibody, small molecule, *etc*.) capable of disrupting the ANLN/RhoA association *in vivo*. The label on the container may indicate that the composition is used for treating one or more conditions characterized by abnormal cell proliferation. The label may also indicate directions for administration and monitoring techniques, such as those described herein.

In addition to the container described above, a treatment kit may optionally comprise a second container housing a pharmaceutically-acceptable diluent. It may further include other materials desirable from a commercial and user standpoint, including other buffers, diluents, filters, needles, syringes, and package inserts with instructions for use.

The compositions may, if desired, be presented in a pack or dispenser device which may contain one or more unit dosage forms containing the active ingredient. The pack may, for example, comprise metal or plastic foil, such as a blister pack. The pack or dispenser device may be accompanied by instructions for administration. Compositions comprising a compound formulated in a compatible pharmaceutical carrier may also be prepared, placed in an appropriate container, and labeled for treatment of an indicated condition.

### VI. Diagnosis and prognosis of cancer

The present methods can be used in the diagnosis, prognosis, classification, and treatment of a number of types of cancers. A cancer at any stage of progression can be detected, such as primary, metastatic, and recurrent cancers. Exemplary cancers for diagnosis, prognosis, classification include, *e*.*g*., lung cancers such as non-small cell lung cancer (NSCLC).

The present invention provides methods for determining the prognosis of mammals with NSCLC. Such methods are based on the discovery that nuclear localization of ANLN occurs most frequently in those non-small cell lung cancer patients with a poor prognosis. Accordingly, by determining whether or not a sample (*e*.*g*., a biopsy) from an individual comprises cells in which ANLN is localized to the nucleus rather than the cytoplasm, it is possible to predict the prognosis of the individual.

As described herein, localization of ANLN may occur completely or nearly completely in the cytoplasm, or may be localized in both the nucleus and cytoplasm of a cell. "Nuclear localization" thus encompasses situations where ANLN is detectable in both the cytoplasm and nucleus.

Nuclear localization may be determined by any method known in the art. In some embodiments, immunohistochemical analyses are used to detect ANLN in cells in tissue or cytological samples obtained by surgery or the minimally invasive techniques available at every hospital such as sputum test or any biopsies. Determination of a poor prognosis may be used to determine further treatment, *e*.*g*., to stop further treatments that reduce quality of life or to treat the cancer in a different manner than previously used or to treat the cancer more aggressively. Namely, the prediction of prognosis by ANLN should eventually enable clinicians to choose in advance the most appropriate treatment for each cancer patient without even the information of conventional clinical staging of the disease, using only routine procedures for tissue-sampling.

Further, the present methods may be used to assess the efficacy of a course of treatment. For example, in a mammal with cancer from which a biological sample has been found to contain nuclear-localized ANLN, the efficacy of an anti-cancer treatment can be assessed by monitoring nuclear localization of ANLN over time. For example, a reduction in ANLN localization in a biological sample taken from a mammal following a treatment, compared to a level in a sample taken from the mammal before, or earlier in, the treatment, indicates efficacious treatment.

As noted above, the present invention also describes kits for detecting the cellular location of ANLN. Examples of components of such kits include, immunohistochemical reagents, *e*.*g*., an antibody that binds to ANLN, and a detectable label for detecting the antibody in a cell.

### EXAMPLES

As can be appreciated from the disclosure provided above, the present invention has a wide variety of applications. Accordingly, the following examples are offered for illustration purposes and are not intended to be construed as a limitation on the invention in any way.

### Example 1- Materials and Methods

### (a) Cell lines and clinical samples

The 23 human lung-cancer cell lines used in the examples herein were as follows: lung adenocarcinoma (ADC); A549, LC319, PC-3, PC-9, PC-14, A427, NCI-H1373, a bronchioloalveolar cell carcinoma (BAC); NCI-H1666, NCI-H1781, lung squamous-cell carcinoma (SCC); RERF-LC-AI, SK-MES-1, EBC-1, LU61, NCI-H520, NCI-H1703, NCI-H2170, lung adenosquamous carcinoma (ASC); NCI-H226, NCI-H647, lung large-cell carcinoma (LCC); LX1, small-cell lung cancer (SCLC); DMS114, DMS273, SBC-3, SBC-5. All cells were grown in monolayers in appropriate medium supplemented with 10% fetal calf serum (FCS) and were maintained at 37°C in an atmosphere of humidified air with 5% CO₂. Human small airway epithelial cells (SAEC) were used as normal control and were grown in optimized medium (SAGM) purchased from Cambrex Bio Science Inc. (Walkersville, MD). Primary NSCLC samples, of which 22 were classified as ADCs, 14 as SCCs, and one as ASC, had been obtained earlier with informed consent from 37 patients (Kikuchi, T., et al., Oncogene. 22:2192-205 (2003)).

A total of 285 formalin-fixed primary NSCLCs (stage I-IIIA) and adjacent normal lung tissue samples used for immunostaining on tissue microarray were obtained from patients who underwent surgery with informed consent.

### (b) Selection of a candidate gene and analysis by semi-quantitative RT-PCR

On the basis of the gene-expression profile analysis, genes that showed expression levels of 5-fold or higher than normal lung in more than 50% of the tumors were selected for examination. The *ANLN* transcript was contained in a list of the genes and was subsequently confirmed to be over-expressed by semi-quantitative RT-PCR. Appropriate dilutions were prepared of each single-stranded cDNA prepared from mRNA of clinical lung-cancer cells by the use of the β-actin (*ACTB*) expression level as a quantitative control. The primer sets for amplification were:
*ACTB*-F (5'-GAGGTGATAGCATTGCTTTCG-3' (SEQ ID No.5)), and
*ACTB*-R (5'-CAAGTCAGTGTACAGGTAAGC-3' (SEQ ID No.6)) for *ACTB,* and
*ANLN*-F1 (5'-GCTGCGTAGCTTACAGACTTAGC-3' (SEQ ID No.7)), and
*ANLN*-R1 (5'-AAGGCGTTTAAAGGTG ATAGGTG-3' (SEQ ID No.8)) for *ANLN.*

All reactions involved initial denaturation at 94°C for 2 min followed by 21 (for *ACTB*) or 30 cycles (for *ANLN*) of 95°C for 30s, 58-62°C for 30s, and 72°C for 45s on a GeneAmp PCR system 9700 (Applied Biosystems, Foster City, CA).

### (c) Northern-blot analysis

Human multiple-tissue blots (BD Biosciences Clontech, Palo Alto, CA) were hybridized with a ³²P-labeled PCR product of *ANLN.* The full-length cDNA of *ANLN* was prepared by RT-PCR using primers;
*ANLN*-F2 (5'- CCCAAGCTTGGGGCCACCATGGATCCGTTTACGGAGAAAC -3' (SEQ ID No.9)) and
*ANLN*-R2 (5'- TGCTCTAGAGCAAGGCTTTCCAATAGGTTTGTAG -3' (SEQ ID No. 10)).
Pre-hybridization, hybridization, and washing were performed according to the supplier's recommendations. The blots were auto-radiographed with intensifying screens at room temperature for 96 hours.

### (d) Western-blot analysis

Rabbit antibodies specific for human ANLN protein were raised by immunization of rabbits with GST-fused human ANLN protein at codon position 428 - 718. The antibodies were purified using standard protocols. An ECL Western Blotting System (Amersham Biosciences, Uppsala, Sweden) was used. Cells were maintained in serum-free medium for 24 hours after plasmid transfection and were lysed in appropriate amounts of lysing buffer (150 mM NaCl, 50 mM Tris-HCl, pH 8.0, 1% Nonidet P-40, 0.1% SDS, 0.5% deoxychorate-Na, plus protease inhibitor). SDS-PAGE was performed and PAGE-separated proteins were electroblotted onto nitrocellulose membranes (Amersham Biosciences) and incubated with antibodies. A sheep anti-mouse IgG-HRP antibody (Amersham Biosciences) and a goat anti-rabbit IgG-HRP antibody (Amersham Biosciences) were served as the secondary antibodies for these experiments.

### (e) Immunocytochemical analysis

Cultured cells were washed twice with PBS(-), fixed in 4% paraformaldehyde solution for 60 min at room temperature, and then rendered permeable with PBS(-) containing 0.1% Triton X-100 for 1.5 min. Prior to the primary antibody reaction, cells were covered with blocking solution (3% BSA in PBS(-)) for 60 min to block non-specific antibody binding. Then the cells were incubated with antibodies to human ANLN. Antibodies were stained with a goat anti-rabbit secondary antibody conjugated to FITC (Cappel, Durham, NC, USA) or rhodamine (Cappel) for revealing endogenous ANLN, and viewed with a laser-confocal microscopy (TSC SP2 AOBS: Leica Microsystems, Wetzlar, Germany). In order to visualize actin filaments, Alexa594-conjugated phalloidin (Molecular Probes, Eugene, OR, USA) was also added after the incubation with secondary antibodies.

### (f) Antisense S-oligonucleotides

2 X 10⁵ cells of NSCLC cell line A549 that were plated onto 6-well dishes were transfected with synthetic S-oligonucleotides (0.2 µM) corresponding to the *ANLN* gene, using Lipofectamine reagent (40 nM) (Invitrogen, Carlsbad, CA, USA), and maintained for two days in media containing 10% FCS. Cell viability was evaluated by MTT assay, each in triplicate. The sequences of the S-oligonucleotides were as follows: antisense 1 (AS1), 5'-CTCCGTAAACGGATCCAT-3' (SEQ ID No. 11); reverse 1 (R1), 5'-TACCTAGGCAAATGCCTC-3' (SEQ ID No. 12), antisense 2 (AS2), 5'-CGGATCCATCGCCCCAGG-3' (SEQ ID No. 13); reverse 2 (R2), 5'-GGACCCCGCTACCTAGGC-3' (SEQ ID No. 14). MTT assays were performed as described elsewhere (Suzuki, C., et al., Cancer Res. 63:7038-41 (2003)).

### (g) RNA interference assay

A vector-based RNA interference (RNAi) system, psiH1BX3.0, was established to direct the synthesis of siRNAs in mammalian cells, as reported elsewhere (Shimokawa T, et al., Cancer Res. 2003;63:6116-20.). 10 µg of siRNA-expression vector was transfected using 30 µl of Lipofectamine 2000 (Invitrogen) into NSCLC cell lines, LC319 and A549. In this assay, more than 90% of the transfected cells expressed this synthetic siRNA, and endogenous expression of ANLN was effectively suppressed. The transfected cells were cultured for five days in the presence of appropriate concentrations of geneticin (G418). Cell numbers and viability were measured by Giemsa staining and MTT assay in triplicate. The target sequences of the synthetic oligonucleotides for RNAi were as follows: control (LUC (Luciferase: Photinus pyralis luciferase gene), 5'-CGTACGCGGAATACTTCGA-3' (SEQ ID No. 15); SCR (Scramble: Chloroplast Euglena gracilis gene coding for the SS and 16S rRNA), 5'-GCGCGCTTTGTAGGATTCG-3' (SEQ ID No.16)); siRNA-*ANLN*-1 (ski-1), 5'-CCAGTTGAGTCGACATCTG-3' (SEQ ID No. 17); siRNA-*ANLN*-2 (si-2), 5'-GCAGCAGATACCATCAGTG-3' (SEQ ID No.18). To validate our RNAi system, individual control siRNAs were tested by semi-quantitative RT-PCR to confirm the decrease in the expression of the corresponding target-genes that had been transiently transfected to COS-7 cells. Down-regulation of the *ANLN* expression by functional siRNA, but not by controls was also confirmed in the cell lines used for this assay.

### (h) Flow cytometry

Cells were plated at a density of 5 X 10⁵cells/100-mm dish, transfected with siRNA-expression vectors, and cultured in the presence of appropriate concentrations of geneticin. Five days after transfection, cells were trypsinized, collected in PBS, and fixed in 70% cold ethanol for 30 min. After treatment with 100 µg/ml RNase (Sigma-Aldrich Co.), the cells were stained with 50 µg/ml propidium iodide (Sigma-Aldrich Co.) in PBS. Flow cytometry was performed on a Becton Dickinson FACScan and analyzed by ModFit software (Verity Software House, Inc., Topsham, ME, USA). The cells determined from at least 20,000 ungated cells were analyzed for DNA content.

### (i) BrdU incorporation assay

Lung-cancer cells (LC319 and A549 cells) transfected with the plasmids designed to express ANLN and mock plasmids were cultured in serum-free medium for 4 hours. The medium was then replaced by RPMI1640 containing 10% FCS with 10 µM BrdU. These cells were incubated for 20 hours, additionally fixed, and served for measuring incorporated BrdU using a commercially available kit (Cell Proliferation ELISA, BrdU; Roche Diagnostics, Basel, Switzerland).

### (j) Matrigel invasion assay

NIH3T3 and COS-7 cells transfected with plasmids designed to express ANLN or mock plasmids were grown to the confluent stage in DMEM containing 10% FCS. The cells were harvested by trypsinization and subsequently washed in DMEM without addition of serum or proteinase inhibitor. The cells were suspended in DMEM at 1 x 10⁵/ml. Before preparing the cell suspension, the dried layer of Matrigel matrix (Becton Dickinson Labware, Bedford, MA, USA) was rehydrated with DMEM for 2 hours at room temperature. DMEM (0.75 ml) containing 10% FCS was added to each lower chamber of 24-well Matrigel invasion chambers, and 0.5 ml (5 x 10⁴ cells) of cell suspension was added to each insert of the upper chamber. The plates of inserts were incubated for 22 hours at 37°C. After incubation the chambers were processed and the cells invading through the Matrigel-coated inserts were fixed and stained by Giemsa as directed by the supplier (Becton Dickinson Labware).

### (k) Wound migration assay

NIH3T3 cells transfected with plasmids designed to express ANLN or mock plasmids were suspended in serum-free DMEM and plated in individual wells of two well chambers (Becton Dickinson Labware) that had been coated with 10 µg/ml of fibronectin. After 4 hours of incubation, a line of adherent cells were scraped from the bottom of each chamber with a P200 pipette tip to generate wounds, and the medium was replaced with DMEM containing 10% FCS. Cells were allowed to proliferate and migrate into the wound area for 48 hours. Then the number of the cells in the wound area was counted with a microscope (DP50, OLYMPUS, Tokyo, Japan).

### (I) Detection of RHO activation

RHO activation by ANLN was detected using EZ-Detect^{™} Rho Activation Kit (PIERCE, Rockford, IL, USA). Briefly, LC319 cells transfected with ANLN-expressing plasmids or mock plasmids were cultured for 24 hours. Then cells were washed and lysed with lysis buffer. After centrifugation at 16,000xg, the lysate was mixed with GST-rhotekin (RTKN)-RBD and "SwellGel Immobilized Glutathione Disc" for affinity precipitation of activated RHO. The GST-pulled-down precipitant that contained activated RHO was washed and boiled with sample buffer and served for western-blot analysis using anti-RHO (-A, -B, and -C) and anti-ANLN antibodies.

### (m) Immunohistochemistry and tissue microarray

The tumor tissue microarrays using formalin-fixed lung cancers were constructed as published previously (Kononen, J., et al., Nat. Med. 4: 844-847 (1998); Chin, S. F., et al., Mol. Pathol., 56:275-79 (2003); Callagy, G., et al., Diagn. Mol. Pathol., 12:27-34 (2003)). The tissue area for sampling was selected based on a visual alignment with the corresponding HE-stained section on a slide. Three, four, or five tissue cores (diameter 0.6 mm; height 3-4 mm) taken from the donor tumor blocks were placed into a recipient paraffin block using a tissue microarrayer (Beecher Instruments, Hummingbird Court Sun Prairie, WI, USA). A core of normal tissue was also punched out from each case. 5-µm sections of the resulting microarray block were used for immunohistochemical analysis. The staining pattern of ANLN was assessed semi-quantitatively as absent or positive as well as qualitatively according to nuclear or cytoplasmic ANLN (n-ANLN, c-ANLN) by three independent investigators without prior knowledge of the clinical follow-up data. Cases with less than 10% of n- or c-ANLN-stained tumor cells were judged as each type of ANLN negative. Cases were accepted only as positive if reviewers independently defined them thus.

To investigate the presence of ANLN protein in clinical samples on the tissue microarray, the sections were stained using ENVISION+ Kit/horseradish peroxidase (HRP) (DakoCytomation, Glostrup, Denmark). Briefly, anti-human ANLN antibody was added after blocking endogenous peroxidase and proteins, and the sections were incubated with HRP-labeled anti-rabbit IgG as the secondary antibody. Substrate-chromogen was added and the specimens were counterstained with hematoxylin.

### (n) Statistical analysis

The clinicopathological variables such as age, gender, and pathological-TNM stage were examined for their correlation with the expression level of ANLN protein determined by tissue microarray analysis. Tumor specific survival curves were calculated from the date of surgery to the time of death related to NSCLC or time of last follow-up observation. Kaplan-Meier curves were calculated for each relevant variable and for ANLN expression, respectively. Differences in survival times between patient subgroups were analyzed using the Log-rank test. Univariate and multivariate analyses were performed with the Cox proportional hazard regression model to determine the association between clinicopathological variables and cancer-related mortality. First, the association between possible prognostic factors including age, gender, pT-classification, and pN-classification, and death were analyzed, taking into consideration 1 factor at a time. Second, multivariate Cox analysis was performed on backward (stepwise) procedures that always forced ANLN expression into the model, along with any and all variables that satisfied an entry level of *P*-value < 0.05. As the model continued to add factors, independent factors did not exceed an exit level of *P*-value of <0.05.

### Example 2 - Over-expression of ANLN in

### NSCLC tissues and cell lines and normal tissues

Genes that showed 5-fold or higher expression in more than 50% of 37 NSCLCs analyzed by cDNA microarray were previously screened (Kikuchi, T. et al. Oncogene 22(14):2192-2205 (2003)). Among 23,040 genes screened, the *ANIN* transcript was identified as over-expressed frequently in NSCLCs, and confirmed its over-expression in twelve representative NSCLC cases by semi-quantitative RT-PCR experiments (**Fig. 1a**). In addition, high level of *ANLN* (Genbank Accession No.NM_018685) expression was observed in all of 23 lung-cancer cell lines, whereas no PCR product was detected in normal small airway epithelia derived cells (SAEC) (**Fig. 1b**). Northern blotting analysis using *ANLN* cDNA as a probe identified an about 4.0-kb transcript as a weak band, only seen in testis and spinal cord, among the 24 normal human tissues examined (**Fig. 1c**). Expression of ANLN protein in NSCLC cell lines A549, LC319, and NCI-H522 was confirmed by examining endogenous expression of ANLN protein by Western-blotting analysis using anti-ANLN antibody.

### Example 3 - Subcellular localization of ANLN and actin stress fiber formation

To confirm the subcellular localization of ANLN in lung-cancer cells, immunocytochemical analysis was performed. Endogenously expressed ANLN in lung-cancer cell lines LC319 and A549 showed a various subcellular localization pattern (**Fig. 2a**). ANLN protein was observed in the nuclei and/or cytoplasm (n-ANLN and c-ANLN), and in the cortex following nuclear envelope breakdown, the cleavage furrow during cytokinesis, and the midbody at late telophase. Fiber-like staining in cytoplasm was also observed in considerable number of the cells. The co-localization of endogenous ANLN and F-actin on stress fibers in these cells was confirmed by immunostaining using anti-ANLN antibody and phalloidin (**Fig. 2b**). Since the actin cytoskeleton is known to play an important role in cytokinesis and morphology of mammalian cells, the effect of ANLN on actin-stress fiber formation was next examined by transfection of ANLN- expressing plasmids into LC319 and NIH3T3 cells. Fiber-like staining detected with phalloidin was significantly increased after transfection of ANLN, suggesting that over-expression of exogenous ANLN induced many stress fibers in these cells (**Fig. 2c**).

### Example 4 - Effects of ANLN on growth of NSCLC cells

To assess whether ANLN is essential for growth or survival of lung-cancer cells, two pairs of reverse (control) and antisense S-oligonucleotides corresponding to the ANLN sequence (Genbank Accession No.NM_018685, SEQ ID NO:1, encoding SEQ ID NO:2) were synthesized and each transfected into A549 cells that had shown a high level of *ANLN* expression. Introduction of each of the two different antisense S-oligonucleotides (AS1 and AS2) decreased cell viability compared with the corresponding control nucleotides (R1 and R2), suggesting that *ANLN* was essential to the growth and/or survival of the cancer cells (data not shown). To further confirm that the growth suppressive effect by antisense S-oligonucleotides was *ANLN*-specific, plasmids were designed and constructed to express siRNA against *ANLN* (siRNA-*ANLN*-1 and -2), and two control plasmids (siRNAs for Luciferase (LUC), or Scramble (SCR)), and transfected each of them into LC319 and A549 cells. The amount of *ANLN* transcript in the cells transfected with siRNA-*ANLN*-1 or -2 was significantly decreased in comparison with those transfected with either of the two control siRNAs (**Fig. 3a**); transfection of siRNA-*ANLN*-1 or -2 also resulted in significant decreases in colony numbers and cell viability measured by colony-formation and MTT assays (**Fig. 3b, c**). Moreover, the cells treated with siRNA-*ANLN*-1 showed larger cell morphology with multiple nuclei (**Fig. 3d**). To clarify the molecular mechanisms of this phenotype further, flow cytometry was performed using LC319 cells that had been transfected with siRNA-*ANLN*-1 and found that the proportion of cells with a DNA content of 4N-16N in the cells transfected with siRNA-*ANLN*-1 was significantly higher than that in the cells transfected with control siRNA (LUC) (**Fig. 3e**).

To further investigate the effects of ANLN on the regulation of cell cycle progression, BrdU incorporation assays were performed using LC319 and A549 cells transiently transfected with ANLN-expressing plasmids. DNA synthesis was likely to be enhanced by the induction of ANLN expression in a dose dependent manner in both cell lines (**Fig. 4a**).

### Example 5 - Effect of ANLN on cellular motility

As the immunocytochemical analysis indicated that ANLN protein and F-actin on stress fibers were co-localized and induction of exogenous ANLN expression promoted the formation of actin stress fibers in mammalian cells, Matrigel invasion assays were subsequently performed to determine whether ANLN could play a possible role in cellular motility. Invasion of NIH3T3 and COS-7 cells transfected with ANLN expression vectors through Matrigel were significantly promoted, compared to the control cells (**Fig. 4b, 4c**). Wound migration assay using NIH3T3 cells transfected with plasmids designed to express ANLN or mock plasmids also showed that migration of the ANLN-expressing cells were significantly activated.

To clarify the mechanism of activation of cellular motility by ANLN, the following assays were carried out. Since the small GTPase RhoA was known to control the formation of actin structures, the possible interaction between RhoA and ANLN was first examined. As shown in Figure 5a, the co-localization of endogenous RhoA and ANLN in cytoplasm and in the cleavage furrow of the lung-cancer cell line, LC319 cells was detected by immunocyotochemical analysis. The direct association of endogenous RhoA with exogenously expressed ANLN was confirmed by immunoprecipitation assays (**Fig. 5b**).

Like other GTPases, RHO is active when bound to GTP and inactive when bound to GDP. Upon binding to GTP, RHO interacts with downstream effectors such as rhotekin (RTKN). Based on these facts, the interaction of ANLN with active form of RHO was investigated. Specifically, a GST-pull-down assay was performed using GST-fusion RTKN to affinity-precipitate the complex containing GTP-RHO (active form) and ANLN; the immune-complex containing ANLN and RHO was detected by Western blotting analysis using of either of the antibodies. When LC319 cells were transfected with plasmids designed to express ANLN, the induction of RHO activation as well as the direct interaction between ANLN and the active form of RHO was observed (**Fig. 5c**, *upper and lower panels*).

### Example 6 - Nuclear ANLN expression is associated with a poor prognosis

Immunohistochemical analysis was performed with an anti-ANLN polyclonal antibody using tissue microarrays that consisted of 285 NSCLC tissues, all of which were resected surgically. The study showed that 267 (93.6%) of the 285 cases were positively stained only with c-ANLN (cytoplasmic), 128 (44.9%) were positive with both c- ANLN and n-ANLN (nuclear), and no case was positive with n-ANLN alone (**Fig. 6a**). The question then was whether or not ANLN expression was associated with clinical outcome of NSCLC. Statistical analysis revealed no significant correlation of c- or n-ANLN expression with pT- or pN-factors. However, a striking association was found between n-ANLN expression and tumor-specific 5 year-survival using Kaplan-Meier method (*P* < 0.0001 by the Log-rank test) (**Fig. 6b**). Using univariate analysis, pT, pN, gender, and n-ANLN expression were significantly related to a poor tumor-specific survival of NSCLC patients. Furthermore, n-ANLN staining was determined to be an independent prognostic factor by multivariate analysis using Cox proportional hazard model (*P* = 0.001).

### Industrial Applicability

As demonstrated herein, ANLN interacts with RhoA, and the inhibition of the interaction leads to the inhibition of cell proliferation of cancer cells. Thus, agents that inhibit the binding of ANLN and RhoA and prevent its activity may find therapeutic utility as anti-cancer agents, particularly anti-cancer agents for the treatment of lung cancers such as NSCLC.

## Claims

1. A method of predicting a non-small cell lung cancer (NSCLC) prognosis in a subject, wherein the method comprises the steps of:
(a) detecting an ANLN protein localized in the nucleus of a specimen collected from the subject whose NSCLC prognosis is to be predicted; and
(b) predicting a poor prognosis when localization of the ANLN protein in the nucleus is detected.

2. The method of claim 1, wherein the localization of ANLN in the nucleus of the specimen is detected by:
(a) contacting an antibody recognizing the ANLN protein with the specimen; and
(b) detecting the antibody which binds to the specimen in the nuclear region.

## Patentansprüche

1. Verfahren zur Vorhersage einer Prognose eines nicht-kleinzelligen Lungenkrebses (NSCLC) in einem Individuum, wobei das Verfahren die Schritte umfasst:
(a) Nachweis eines ANLN-Proteins, welches in dem Zellkern einer Probe lokalisiert ist, die von dem Individuum, dessen NSCLC-Prognose vorherzusagen ist, gewonnen wurde; und
(b) Vorhersagen einer schlechten Prognose, falls die Lokalisation des ANLN-Proteins in dem Zellkern nachgewiesen wird.

2. Verfahren nach Anspruch 1, wobei die Lokalisation von ANLN in dem Zellkern der Probe nachgewiesen wird durch:
(a) Inkontaktbringen eines Antikörpers; welcher das ANLN-Protein in der Probe erkennt; und
(b) Nachweis des Antikörpers, welcher in der Zellkernregion der Probe bindet.

## Revendications

1. Méthode pour prédire le pronostic d'un cancer du poumon non à petites cellules (CPNPC) chez un sujet, dans laquelle la méthode comprend les étapes de :
(a) détection de la protéine ANLN localisée dans le noyau d'un spécimen recueilli d'un sujet dont le pronostic du CPNPC est à prédire ; et
(b) prédire un mauvais pronostic quand la localisation de la protéine ANLN est détectée dans le noyau.

2. Méthode de la revendication 1, dans laquelle la localisation de ANLN dans le noyau du spécimen est détectée par :
(a) la mise en contact d'un anticorps reconnaissant la protéine ANLN avec le spécimen ; et
(b) la détection de l'anticorps qui se lie au spécimen dans la région nucléaire.


### SEQUENCE LISTING

<110> ONCOTHERAPY SCIENCE, INC.
   THE UNIVERSITY OF TOKYO
<120> NON-SMALL CELL LUNG CANCER-RELATED GENE, ANLN, AND ITS INTERACTIO NS WITH RhoA
<130> ONC-A0408P
<150> US 60/600,561
   <151> 2004-08-10
<160> 18
<170> PatentIn version 3.1
<210> 1
   <211> 4786
   <212> DNA
   <213> Homo sapiens
<220>
   <221> CDS
   <222> (205).. (3579)
   <223>
<400> 1
ctcggcgctg aaattcaaat ttgaacggct gcagaggccg agtccgtcac tggaagccga      60
   gaggagagga cagctggttg tgggagagtt cccccgcctc agactcctgg ttttttccag      120
   gagacacact gagctgagac tcacttttct cttcctgaat ttgaaccacc gtttccatcg     180
   tctcgtagtc cgacgcctgg ggcg atg gat ccg ttt acg gag aaa ctg ctg       231
   gag cga acc cgt gcc agg cga gag aat ctt cag aga aaa atg gct gag      279
   agg ccc aca gca gct cca agg tct atg act cat gct aag cga gct aga      327
   cag cca ctt tca gaa gca agt aac cag cag ccc ctc tct ggt ggt gaa      375
   gag aaa tct tgt aca aaa cca tcg cca tca aaa aaa cgc tgt tct gac      423
   aac act gaa gta gaa gtt tct aac ttg gaa aat aaa caa cca gtt gag 471
   tcg aca tct gca aaa tct tgt tct cca agt cct gtg tct cct cag gtg 519
   cag cca caa gca gca gat acc atc agt gat tct gtt gct gtc ccg gca 567
   tca ctg ctg ggc atg agg aga ggg ctg aac tca aga ttg gaa gca act 615
   gca gcc tcc tca gtt aaa aca cgt atg caa aaa ctt gca gag caa cgg 663
   cgc cgt tgg gat aat gat gat atg aca gat gac att cct gaa agc tca 711
   ctc ttc tca cca atg cca tca gag gaa aag gct gct tcc cct ccc aga 759
   cct ctg ctt tca aat gcc tcg gca act cca gtt ggc aga agg ggc cgt 807
   ctg gcc aat ctt gct gca act att tgc tcc tgg gaa gat gat gta aat 855
   cac tca ttt gca aaa caa aac agt gta caa gaa cag cct ggt acc gct 903
   tgt tta tcc aaa ttt tcc tct gca agt gga gca tct gct agg atc aat 951
   agc agc agt gtt aag cag gaa gct aca ttc tgt tcc caa agg gat ggc 999
   gat gcc tct ttg aat aaa gcc cta tcc tca agt gct gat gat gcg tct 1047
   ttg gtt aat gcc tca att tcc agc tct gtg aaa gct act tct cca gtg 1095
   aaa tct act aca tct atc act gat gct aaa agt tgt gag gga caa aat 1143
   cct gag cta ctt cca aaa act cct att agt cct ctg aaa acg ggg gta 1191
   tcg aaa cca att gtg aag tca act tta tcc cag aca gtt cca tcc aag 1239
   gga gaa tta agt aga gaa att tgt ctg caa tct caa tct aaa gac aaa 1287
   tct acg aca cca gga gga aca gga att aag cct ttc ctg gaa cgc ttt 1335
   gga gag cgt tgt caa gaa cat agc aaa gaa agt cca gct cgt agc aca 1383
   ccc cac aga acc ccc att att act cca aat aca aag gcc atc caa gaa 1431
   aga tta ttc aag caa gac aca tct tca tct act acc cat tta gca caa 1479
   cag ctc aag cag gaa cgt caa aaa gaa cta gca tgt ctt cgt ggc cga 1527
   ttt gac aag ggc aat ata tgg agt gca gaa aaa ggc gga aac tca aaa 1575
   agc aaa caa cta gaa acc aaa cag gaa act cac tgt cag agc act ccc 1623
   ctc aaa aaa cac caa ggt gtt tca aaa act cag tca ctt cca gta aca 1671
   gaa aag gtg acc gaa aac cag ata cca gcc aaa aat tct agt aca gaa 1719
   cct aaa ggt ttc act gaa tgc gaa atg acg aaa tct agc cct ttg aaa 1767
   ata aca ttg ttt tta gaa gag gac aaa tcc tta aaa gta aca tca gac 1815
   cca aag gtt gag cag aaa att gaa gtg ata cgt gaa att gag atg agt 1863
   gtg gat gat gat gat atc aat agt tcg aaa gta att aat gac ctc ttc 1911
   agt gat gtc cta gag gaa ggt gaa cta gat atg gag aag agc caa gag 1959
   gag atg gat caa gca tta gca gaa agc agc gaa gaa cag gaa gat gca 2007
   ctg aat atc tcc tca atg tct tta ctt gca cca ttg gca caa aca gtt 2055
   ggt gtg gta agt cca gag agt tta gtg tcc aca cct aga ctg gaa ttg 2103
   aaa gac acc agc aga agt gat gaa agt cca aaa cca gga aaa ttc caa 2151
   aga act cgt gtc cct cga gct gaa tct ggt gat agc ctt ggt tct gaa 2199
   gat cgt gat ctt ctt tac agc att gat gca tat aga tct caa aga ttc 2247
   aaa gaa aca gaa cgt cca tca ata aag cag gtg att gtt cgg aag gaa 2295
   gat gtt act tca aaa ctg gat gaa aaa aat aat gcc ttt cct tgt caa 2343
   gtt aat atc aaa cag aaa atg cag gaa ctc aat aac gaa ata aat atg 2391
   caa cag aca gtg atc tat caa gct agc cag gct ctt aac tgc tgt gtt 2439
   gat gaa gaa cat gga aaa ggg tcc cta gaa gaa gct gaa gca gaa aga 2487
   ctt ctt cta att gca act ggg aag aga aca ctt ttg att gat gaa ttg 2535
   aat aaa ttg aag aac gaa gga cct cag agg aag aat aag gct agt ccc 2583
   caa agt gaa ttt atg cca tcc aaa gga tca gtt act ttg tca gaa atc 2631
   cgc ttg cct cta aaa gca gat ttt gtc tgc agt acg gtt cag aaa cca 2679
   gat gca gca aat tac tat tac tta att ata cta aaa gca gga gct gaa 2727
   aat atg gta gcc aca cca tta gca agt act tca aac tct ctt aac ggt 2775
   gat gct ctg aca ttc act act aca ttt act ctg caa gat gta tcc aat 2823
   gac ttt gaa ata aat att gaa gtt tac agc ttg gtg caa aag aaa gat 2871
   ccc tca ggc ctt gat aag aag aaa aaa aca tcc aag tcc aag gct att 2919
   act cca aag cga ctc ctc aca tct ata acc aca aaa agc aac att cat 2967
   tct tca gtc atg gcc agt cca gga ggt ctt agt gct gtg cga acc agc 3015
   aac ttc gcc ctt gtt gga tct tac aca tta tca ttg tct tca gta gga 3063
   aat act aag ttt gtt ctg gac aag gtc ccc ttt tta tct tct ttg gaa 3111
   ggt cat att tat tta aaa ata aaa tgt caa gtg aat tcc agt gtt gaa 3159
   gaa aga ggt ttt cta acc ata ttt gaa gat gtt agt ggt ttt ggt gcc 3207
   tgg cat cga aga tgg tgt gtt ctt tct gga aac tgt ata tct tat 3252
   tgg act tat cca gat gat gag aaa cgc aag aat ccc ata gga agg 3297
   ata aat ctg gct aat tgt acc agt cgt cag ata gaa cca gcc aac 3342
   aga gaa ttt tgt gca aga cgc aac act ttt gaa tta att act gtc 3387
   cga cca caa aga gaa gat gac cga gag act ctt gtc agc caa tgc 3432
   agg gac aca ctc tgt gtt acc aag aac tgg ctg tct gca gat act 3477
   aaa gaa gag cgg gat ctc tgg atg caa aaa ctc aat caa gtt ctt 3522
   gtt gat att cgc ctc tgg caa cct gat gct tgc tac aaa cct att 3567
   gga aag cct taa accgggaaat ttccatgcta tctagaggtt tttgatgtca 3619
   tcttaagaaa cacacttaag agcatcagat ttactgattg cattttatgc tttaagtacg 3679
   aaagggtttg tgccaatatt cactacgtat tatgcagtat ttatatcttt tgtatgtaaa 3739
   actttaactg atttctgtca ttcatcaatg agtagaagta aatacattat agttgatttt 3799
   gctaaatctt aatttaaaag cctcattttc ctagaaatct aattattcag ttattcatga 3859
   caatattttt ttaaaagtaa gaaattctga gttgtcttct tggagctgta ggtcttgaag 3919
   cagcaacgtc tttcaggggt tggagacaga aacccattct ccaatctcag tagttttttc 3979
   gaaaggctgt gatcatttat tgatcgtgat atgacttgtt actagggtac tgaaaaaaat 4039
   gtctaaggcc tttacagaaa catttttagt aatgaggatg agaacttttt caaatagcaa 4099
   atatatattg gcttaaagca tgaggctgtc ttcagaaaag tgatgtggac ataggaggca 4159
   atgtgtgaga cttgggggtt caatatttta tatagaagag ttaataagca catggtttac 4219
   atttactcag ctactatata tgcagtgtgg tgcacatttt cacagaattc tggcttcatt 4279
   aagatcatta tttttgctgc gtagcttaca gacttagcat attagttttt tctactccta 4339
   caagtgtaaa ttgaaaaatc tttatattaa aaaagtaaac tgttatgaag ctgctatgta 4399
   ctaataatac tttgcttgcc aaagtgtttg ggttttgttg ttgtttgttt gtttgtttgt 4459
   ttttggttca tgaacaacag tgtctagaaa cccattttga aagtggaaaa ttattaagtc 4519
   acctatcacc tttaaacgcc tttttttaaa attataaaat attgtaaagc agggtctcaa 4579
   cttttaaata cactttgaac ttcttctctg aattattaaa gttctttatg acctcattta 4639
   taaacactaa attctgtcac ctcctgtcat tttatttttt attcattcaa atgtattttt 4699
   tcttgtgcat attataaaaa tatattttat gagctcttac tcaaataaat acctgtaaat 4759
   gtctaaagga aaaaaaaaaa aaaaaaa 4786
<210> 2
   <211> 1124
   <212> PRT
   <213> Homo sapiens
<400> 2
<210> 3
   <211> 1777
   <212> DNA
   <213> Homo sapiens
<220>
   <221> CDS
   <222> (152).. (733)
   <223>
<400> 3
   ggctaccctc gccccgcccg cggtcctccg tcggttctct cattagtcca cggtctggtc 60
   ttcagctacc cgccttcgtc tccgagtttg cgactcgcgg gaccggcgtc cccggcgcga 120
   agaggctgga ctcggattcg ttgcctgagc a atg gct gcc atc cgg aag aaa 172
   Met Ala Ala Ile Arg Lys Lys
   1 5
   ctg gtg att gtt ggt gat gga gcc tgt gga aag aca tgc ttg ctc ata 220
   Leu Val Ile Val Gly Asp Gly Ala Cys Gly Lys Thr Cys Leu Leu Ile
   10 15 20
   gtc ttc agc aag gac cag ttc cca gag gtg tat gtg ccc aca gtg ttt 268
   Val Phe Ser Lys Asp Gln Phe Pro Glu Val Tyr Val Pro Thr Val Phe
   25 30 35
   gag aac tat gtg gca gat atc gag gtg gat gga aag cag gta gag ttg 316
   Glu Asn Tyr Val Ala Asp Ile Glu Val Asp Gly Lys Gln Val Glu Leu
   40 45 50 55
   gct ttg tgg gac aca gct ggg cag gaa gat tat gat cgc ctg agg ccc 364
   Ala Leu Trp Asp Thr Ala Gly Gln Glu Asp Tyr Asp Arg Leu Arg Pro
   60 65 70
   ctc tcc tac cca gat acc gat gtt ata ctg atg tgt ttt tcc atc gac 412
   Leu Ser Tyr Pro Asp Thr Asp Val Ile Leu Met Cys Phe Ser Ile Asp
   75 80 85
   agc cct gat agt tta gaa aac atc cca gaa aag tgg acc cca gaa gtc 460
   Ser Pro Asp Ser Leu Glu Asn Ile Pro Glu Lys Trp Thr Pro Glu Val
   90 95 100
   aag cat ttc tgt ccc aac gtg ccc atc atc ctg gtt ggg aat aag aag 508
   Lys His Phe Cys Pro Asn Val Pro Ile Ile Leu Val Gly Asn Lys Lys
   105 110 115
   gat ctt cgg aat gat gag cac aca agg cgg gag cta gcc aag atg aag 556
   Asp Leu Arg Asn Asp Glu His Thr Arg Arg Glu Leu Ala Lys Met Lys
   120 125 130 135
   cag gag ccg gtg aaa cct gaa gaa ggc aga gat atg gca aac agg att 604
   Gln Glu Pro Val Lys Pro Glu Glu Gly Arg Asp Met Ala Asn Arg Ile
   140 145 150
   ggc gct ttt ggg tac atg gag tgt tca gca aag acc aaa gat gga gtg 652
   Gly Ala Phe Gly Tyr Met Glu Cys Ser Ala Lys Thr Lys Asp Gly Val
   155 160 165
   aga gag gtt ttt gaa atg gct acg aga gct gct ctg caa gct aga cgt 700
   Arg Glu Val Phe Glu Met Ala Thr Arg Ala Ala Leu Gln Ala Arg Arg
   170 175 180
   ggg aag aaa aaa tct ggt tgc ctt gtc ttg tga aaccttgctg caagcacagc 753
   Gly Lys Lys Lys Ser Gly Cys Leu Val Leu
   185 190
   ccttatgcgg ttaattttga agtgctgttt attaatctta gtgtatgatt actggccttt 813
   ttcatttatc tataatttac ctaagattac aaatcagaag tcatcttgct accagtattt 873
   agaagccaac tatgattatt aacgatgtcc aacccgtctg gcccaccagg gtccttttga 933
   cactgctcta acagccctcc tctgcactcc cacctgacac accaggcgct aattcaagga 993
   atttcttaac ttcttgcttc tttctagaaa gagaaacagt tggtaacttt tgtcaattag 1053
   gctgtaacta ctttataact aacatgtcct gccctattat ctgtcagctg caaggtactc 1113
   tggtgagtca ccacttcagg gctttactcc gtaacagatt ttgttggcat agctctgggg 1173
   tgggcagttt tgaaaatggg ctcaaccaga aaagcccaag ttcatgcagc tgtggcagag 1233
   ttacagttct gtggtttcat gttagttacc ttatagttac tgtgtaatta gtgccactta 1293
   atgtatgtta ccaaaaataa atatatctac ccagactaga tgtagtattt tgtataattg 1353
   gattctaata ctgtcatctc aagaagtgta tggtttaaag aagtgtattg gaaataaagt 1413
   cagatggaaa ttcattttaa attcccgttt gtcacttttc tgataaaaga tggccatatt 1473
   accccttttc ggccccatgt atctcagtac cccatggagc tgggctaagt aaataggaat 1533
   tggtttcacg cctcaggcaa ttagacactt tggaagatgg cataacctgt ctcacctgga 1593
   cttaagcgtc tggctctaat tcacagtgct ctttctcctc actgtatcca ggttccctcc 1653
   cagaggagcc accagttctc atgggtggca ctcagtctct cttctctcca gctgactaaa 1713
   ctttttttct gtaccagtta atttttccaa ctactaatag aataaaggca gttttctaaa 1773
   aaaa 1777
<210> 4
   <211> 193
   <212> PRT
   <213> Homo sapiens
<400> 4
   Met Ala Ala Ile Arg Lys Lys Leu Val Ile Val Gly Asp Gly Ala Cys
   1 5 10 15
   Gly Lys Thr Cys Leu Leu Ile Val Phe Ser Lys Asp Gln Phe Pro Glu
   20 25 30
   Val Tyr Val Pro Thr Val Phe Glu Asn Tyr Val Ala Asp Ile Glu Val
   35 40 45
   Asp Gly Lys Gln Val Glu Leu Ala Leu Trp Asp Thr Ala Gly Gln Glu
   50 55 60
   Asp Tyr Asp Arg Leu Arg Pro Leu Ser Tyr Pro Asp Thr Asp Val Ile
   65 70 75 80
   Leu Met Cys Phe Ser Ile Asp Ser Pro Asp Ser Leu Glu Asn Ile Pro
   85 90 95
   Glu Lys Trp Thr Pro Glu Val Lys His Phe Cys Pro Asn Val Pro Ile
   100 105 110
   Ile Leu Val Gly Asn Lys Lys Asp Leu Arg Asn Asp Glu His Thr Arg
   115 120 125
   Arg Glu Leu Ala Lys Met Lys Gln Glu Pro Val Lys Pro Glu Glu Gly
   130 135 140
   Arg Asp Met Ala Asn Arg Ile Gly Ala Phe Gly Tyr Met Glu Cys Ser
   145 150 155 160
   Ala Lys Thr Lys Asp Gly Val Arg Glu Val Phe Glu Met Ala Thr Arg
   165 170 175
   Ala Ala Leu Gln Ala Arg Arg Gly Lys Lys Lys Ser Gly Cys Leu Val
   180 185 190
   Leu
<210> 5
   <211> 21
   <212> DNA
   <213> Artificial
<220>
   <223> An artificially synthesized primer sequence for RT-PCR
<400> 5
   gaggtgatag cattgctttc g 21
<210> 6
   <211> 21
   <212> DNA
   <213> Artificial
<220>
   <223> An artificially synthesized primer sequence for RT-PCR
<400> 6
   caagtcagtg tacaggtaag c 21
<210> 7
   <211> 23
   <212> DNA
   <213> Artificial
<220>
   <223> An artificially synthesized primer sequence for RT-PCR
<400> 7
   gctgcgtagc ttacagactt agc 23
<210> 8
   <211> 23
   <212> DNA
   <213> Artificial
<220>
   <223> An artificially synthesized primer sequence for RT-PCR
<400> 8
   aaggcgttta aaggtgatag gtg 23
<210> 9
   <211> 40
   <212> DNA
   <213> Artificial
<220>
   <223> An artificially synthesized primer sequence for RT-PCR
<400> 9
   cccaagcttg gggccaccat ggatccgttt acggagaaac 40
<210> 10
   <211> 34
   <212> DNA
   <213> Artificial
<220>
   <223> An artificially synthesized primer sequence for RT-PCR.
<400> 10
   tgctctagag caaggctttc caataggttt gtag 34
<210> 11
   <211> 18
   <212> DNA
   <213> Artificial
<220>
   <223> An artificially synthesized S-oligonucleotide sequence.
<400> 11
   ctccgtaaac ggatccat 18
<210> 12
   <211> 18
   <212> DNA
   <213> Artificial
<220>
   <223> An artificially synthesized S-oligonucleotide sequence.
<400> 12
   tacctaggca aatgcctc 18
<210> 13
   <211> 18
   <212> DNA
   <213> Artificial
<220>
   <223> An artificially synthesized S-oligonucleotide sequence.
<400> 13
   cggatccatc gccccagg 18
<210> 14
   <211> 18
   <212> DNA
   <213> Artificial
<220>
   <223> An artificially synthesized S-oligonucleotide sequence.
<400> 14
   ggaccccgct acctaggc 18
<210> 15
   <211> 19
   <212> DNA
   <213> Artificial
<220>
   <223> An artificially synthesized target sequence for siRNA.
<400> 15
   cgtacgcgga atacttcga 19
<210> 16
   <211> 19
   <212> DNA
   <213> Artificial
<220>
   <223> An artificially synthesized target sequence for siRNA.
<400> 16
   gcgcgctttg taggattcg 19
<210> 17
   <211> 19
   <212> DNA
   <213> Artificial
<220>
   <223> An artificially synthesized target sequence for siRNA.
<400> 17
   ccagttgagt cgacatctg 19
<210> 18
   <211> 19
   <212> DNA
   <213> Artificial
<220>
   <223> An artificially synthesized target sequence for siRNA.
<400> 18
   gcagcagata ccatcagtg 19